# EUROPEAN PATENT APPLICATION

(11) **EP 1 221 485 A2**
(43) Date of publication of application: **10.07.2002**
(21) Application number: 02002959.1
(22) Date of filing: 09.03.1999
(51) Int. Cl.: C12N 15/30, C12N 15/85, C12N 5/10, C07K 14/44, C07K 16/20, A61K 39/002

(54) **Polynucleotide molecules encoding neospora proteins**

(30) Priority: 26.03.1998 US 79389 P; 15.12.1998 US 112282 P
(62) Divisional of application: 99301746.6
(71) Applicant: Pfizer Products Inc., Groton, Connecticut 06340 (US)
(72) Inventor: Brake, David Alan, East Lyme, Connecticut 06333 (US); Madura (née Coleman), Rebecca Anne, Westerly, Rhode Island 02891 (US); Durtschi, Becky Ann, Ledyard, Connecticut 06339 (US); Krishnan, Balakrishnan Rajendra, East Lyme, Connecticut 06333 (US); Yoder, Susan Christine, Salem, Connecticut 06340 (US)
(74) Representative: Eddowes, Simon

(57) **Abstract**

The present invention provides isolated polynucleotide molecules comprising nucleotide sequences encoding GRA1, GRA2, SAG1, MIC1 and MAG1 proteins from *Neospora caninum*, as well as recombinant vectors, transformed host cells, and recombinantly-expressed proteins. The present invention further provides a polynucleotide molecule comprising the nucleotide sequence of the bidirectional *GRA1*/*MAG1* promoter of *N. caninum.* The present invention further provides genetic constructs based on the polynucleotide molecules of the present invention that are useful in preparing modified strains of *Neospora* cells for use in vaccines against neosporosis.

SEQUENCE LISTING

<110> Pfizer Products Inc.

<120> POLYNUCLEOTIDE MOLECULES ENCODING NEOSPORA PROTEINS

<130> PC9943A

<140> To Be Assigned
<141> 1999-03-25

<150> 60/079,389
<151> 1998-03-26

<150> 60/112,282
<151> 1998-12-15

<160> 34

<170> PatentIn Ver. 2.1

<210> 1
<211> 1265
<212> DNA
<213> Neospora caninum

<220>
<221> CDS
<222> (205)..(777)

<400> 1
gtttcatcgt tgaactgccg aagagcttta tgtttttgcc gcgacttctt tttctctccc 60

ctgaataaat tgtaccgtgg gtggcgtaca cgtctgaaca cgaactcggc tgggtttgct 120

tttgtggacg tgtttttccg gctcaaataa tttcattttc attgttcata cgtgtttgtg 180

atctctttta aggcattcag caag atg gtg cgt gtg agc gct att gtt ggg 231
                            Met Val Arg Val Ser Ala Ile Val Gly
                              1               5

gtt gca gcc tcg gtg gtt ctc tcc ctt tct tcc ggc gtg tac gcg gcc 279
Val Ala Ala Ser Val Val Leu Ser Leu Ser Ser Gly Val Tyr Ala Ala
 10                  15                  20                  25

gag gga gcg gaa aaa ccc ttg gga ggc gaa ggt caa gcg cct acc ttg 327
Glu Gly Ala Glu Lys Pro Leu Gly Gly Glu Gly Gln Ala Pro Thr Leu
                 30                  35                  40

ttg tca atg cta ggt ggc ggg cgc gcg gga agg ggg ttg tca gtc gga 375
Leu Ser Met Leu Gly Gly Gly Arg Ala Gly Arg Gly Leu Ser Val Gly
            45                  50                  55

caa tca gta gac ctt gac ctg atg ggc aga cgc tac cga gtg acc aga 423
Gln Ser Val Asp Leu Asp Leu Met Gly Arg Arg Tyr Arg Val Thr Arg
        60                  65                  70

tcc gag ggt gcg cca gat gtg ctc gag atc tcc gtt ctg gac gcg gat 471
Ser Glu Gly Ala Pro Asp Val Leu Glu Ile Ser Val Leu Asp Ala Asp
    75                  80                  85

ggg aag gct tct cac atc ggc ttt gta agc att ccg gaa gtg atg gac 519
Gly Lys Ala Ser His Ile Gly Phe Val Ser Ile Pro Glu Val Met Asp
90                  95                  100                 105

acc gtg gcg cgc atg cag aag gac gag gga att ttc ctt gat gcg tta 567
Thr Val Ala Arg Met Gln Lys Asp Glu Gly Ile Phe Leu Asp Ala Leu
                110                 115                 120

agt aaa gga gaa aca gta aag gag gcg atc gag gat gtt gct gca gcg 615
Ser Lys Gly Glu Thr Val Lys Glu Ala Ile Glu Asp Val Ala Ala Ala
            125                 130                 135

gaa ggt ctt tct ccc gag cag act gaa aac ctg gag gaa acg gtg gcc 663
Glu Gly Leu Ser Pro Glu Gln Thr Glu Asn Leu Glu Glu Thr Val Ala
        140                 145                 150

gct gta gcg act ctt gtt cgt gac gag atg gaa gtt ctt aaa gat cag 711
Ala Val Ala Thr Leu Val Arg Asp Glu Met Glu Val Leu Lys Asp Gln
    155                 160                 165

gag aag cta gaa gag gat gca gaa aag ctt gcg gga gat tta gaa gct 759
Glu Lys Leu Glu Glu Asp Ala Glu Lys Leu Ala Gly Asp Leu Glu Ala
170                 175                 180                 185

ctt caa ggg caa cat taa tttgcaaagg gattgtcatg tagccatatg 807
Leu Gln Gly Gln His
                190

ttcaatcgcc ctcaaaagtc gactggggtg ttttggcaca tgtctgcagt tggtttggat 867

cgacggcatg ggttagcgat ggagaaaacg gatcgatggt tgacagttgc cgaaggaaat 927

cggttgcgtc gtgtaaggaa agtgtcacgg gggcattgag atttggaggg gctcttgaag 987

ccttcctcgg tggcaccaga ggggcagagc tcaacgcaag cgtggtatat ggagctggag 1047

cagtggccgc aacgcagcag ggcggcgtga attacgttgc gttagtgctg gcgtgaaacg 1107

tcgtgttctc aacccgagta caatgtagtt tcaggtggtc gttgctcgaa tccgtgtgtc 1167

gcgcctgtgt tgtatagtgt ttcgcattat gtggagacgg ggacgttttt aaaaaatcaa 1227

aaatgtgtgg caaacctgaa aaaaaaaaaa aaaaaaaa 1265

<210> 2
<211> 190
<212> PRT
<213> Neospora caninum

<400> 2
Met Val Arg Val Ser Ala Ile Val Gly Val Ala Ala Ser Val Val Leu
1               5                   10                  15
Ser Leu Ser Ser Gly Val Tyr Ala Ala Glu Gly Ala Glu Lys Pro Leu
            20                  25                  30
Gly Gly Glu Gly Gln Ala Pro Thr Leu Leu Ser Met Leu Gly Gly Gly
        35                  40                  45
Arg Ala Gly Arg Gly Leu Ser Val Gly Gln Ser Val Asp Leu Asp Leu
    50                  55                  60
Met Gly Arg Arg Tyr Arg Val Thr Arg Ser Glu Gly Ala Pro Asp Val
65                  70                  75                  80
Leu Glu Ile Ser Val Leu Asp Ala Asp Gly Lys Ala Ser His Ile Gly
                85                  90                  95
Phe Val Ser Ile Pro Glu Val Met Asp Thr Val Ala Arg Met Gln Lys
            100                 105                 110
Asp Glu Gly Ile Phe Leu Asp Ala Leu Ser Lys Gly Glu Thr Val Lys
        115                 120                 125
Glu Ala Ile Glu Asp Val Ala Ala Ala Glu Gly Leu Ser Pro Glu Gln
    130                 135                 140
Thr Glu Asn Leu Glu Glu Thr Val Ala Ala Val Ala Thr Leu Val Arg
145                 150                 155                 160
Asp Glu Met Glu Val Leu Lys Asp Gln Glu Lys Leu Glu Glu Asp Ala
                165                 170                 175
Glu Lys Leu Ala Gly Asp Leu Glu Ala Leu Gln Gly Gln His
            180                 185                 190

<210> 3
<211> 1774
<212> DNA
<213> Neospora caninum

<400> 3
tgctagtact ggcgagtgaa atgcgacgct cactgtagcc tccagataca cgacctgttg 60
cggagctgac gctctcccca ctagagttca tgagcgatgg ggcgatggta gaccaacggt 120
ccctagcgct tcggctgttg cgcggcggct cttaagagcg ggacgaccgc ctttcaggtg 180
aaccgcctag tatcccaagc acacgaacat cccactcatg ggctggcgga actgctcgca 240
gcggttacgc aaacacagtt gcgacgcaat gagccgtctc aagttgctgt cctcgtctca 300
tttcggatcg gttcccaggt cctccggtgc gtctctgtcg gaaggttatt gcaactccgt 360
tctgcgctgg gattagttta aatcatttca ttaatttgca gtttcatcgt tgaactgccg 420
aagagcttta tgtttttgcc gcgacttctt tttctctccc ctgaataaat tgtaccgtgg 480
gtggcgtaca cgtctgaaca cgaactcggc tgggtttgct tttgtggacg tgtttttccg 540
gctcaaataa tttcattttc attgttcata cgtgtttgtg atctctttta aggcattcag 600
caagatggtg cgtgtgagcg ctattgttgg ggttgcagcc tcggtggttc tctccctttc 660
ttccggcgtg tacgcggccg agggagcgga aaaacccttg ggaggcgaag gtcaagcgcc 720
taccttgttg tcaatgctag gtggcgggcg cgcgggaagg gggttgtcag tcggacaatc 780
agtagacctt gacctgatgg gcagacgcta ccgagtgacc agatccgagg gtgcgccaga 840
tgtgctcgag atctcgtaag tagactactg gtgttcaacg aaaaaaaagt acttgcgctg 900
tggaatgtcg tctgtgtgtt agctgcatca tgtgataagc aaacatttgt tttcgagcgt 960
gtgttgtctc gcgtgctttc agcgttctgg acgcggatgg gaaggcttct cacatcggct 1020
ttgtaagcat tccggaagtg atggacaccg tggcgcgcat gcagaaggac gagggaattt 1080
tccttgatgc gttaagtaaa ggagaaacag taaaggaggc gatcgaggat gttgctgcag 1140
cggaaggtct ttctcccgag cagactgaaa acctggagga aacggtggcc gctgtagcga 1200
ctcttgttcg tgacgagatg gaagttctta aagatcagga gaagctagaa gaggatgcag 1260
aaaagcttgc gggagattta gaagctcttc aagggcaaca ttaatttgca aagggattgt 1320
catgtagcca tatgttcaat cgccctcaaa agtcgactgg ggtgttttgg cacatgtctg 1380
cagttggttt ggatcgacgg catgggttag cgatggagaa aacggatcga tggttgacag 1440
ttgccgaagg aaatcggttg cgtcgtgtaa ggaaagtgtc acgggggcat tgagatttgg 1500
aggggctctt gaagccttcc tcggtggcac cagaggggca gagctcaacg caagcgtggt 1560
atatggagct ggagcagtgg ccgcaacgca gcagggcggc gtgaattacg ttgcgttagt 1620
gctggcgtga aacgtcgtgt tctcaacccg agtacaatgt agtttcaggt ggtcgttgct 1680
cgaatccgtg tgtcgcgcct gtgttgtata gtgtttcgca ttatgtgtag acggggacgt 1740
ttttaaaaaa tcaaaaatgt gtggcaaacc tgaa 1774

<210> 4
<211> 1031
<212> DNA
<213> Neospora caninum

<220>
<221> CDS
<222> (25)..(660)

<400> 4
aaataggggt ttcagcacca cacg atg ttc acg ggg aaa cgt tgg ata ctt 51
Met Phe Thr Gly Lys Arg Trp Ile Leu
1               5

gtt gtt gcc gtt ggc gcc ctg gtc ggc gcc tcg gta aag gca gcc gat 99

Val Val Ala Val Gly Ala Leu Val Gly Ala Ser Val Lys Ala Ala Asp
10                  15                  20                  25

ttt tct ggc agg gga acc gtc aat gga cag ccg gtt ggc agc ggt tat    147
Phe Ser Gly Arg Gly Thr Val Asn Gly Gln Pro Val Gly Ser Gly Tyr
                30                  35                  40

tcc gga tat ccc cgt ggc gat gat gtt aga gaa tca atg gct gca ccc    195
Ser Gly Tyr Pro Arg Gly Asp Asp Val Arg Glu Ser Met Ala Ala Pro
            45                  50                  55

gaa gat ctg cca ggc gag agg caa ccg gag aca ccc acg gcg gaa gct    243
Glu Asp Leu Pro Gly Glu Arg Gln Pro Glu Thr Pro Thr Ala Glu Ala
        60                  65                  70

gta aaa cag gca gcg gca aaa gct tat cga tta ctc aag cag ttt act    291
Val Lys Gln Ala Ala Ala Lys Ala Tyr Arg Leu Leu Lys Gln Phe Thr
    75                  80                  85

gcg aag gtc gga cag gaa act gag aac gcc tac tac cac gtg aag aaa    339
Ala Lys Val Gly Gln Glu Thr Glu Asn Ala Tyr Tyr His Val Lys Lys
90                  95                  100                 105

gcg aca atg aaa ggc ttt gac gtt gca aaa gac cag tcg tat aag ggc    387
Ala Thr Met Lys Gly Phe Asp Val Ala Lys Asp Gln Ser Tyr Lys Gly
                110                 115                 120

tac ttg gcc gtc agg aaa gcc aca gct aag ggc ctg cag agc gct ggc    435
Tyr Leu Ala Val Arg Lys Ala Thr Ala Lys Gly Leu Gln Ser Ala Gly
            125                 130                 135

aag agc ctt gag ctt aaa gag tcg gca ccg aca ggc act acg act gcg    483
Lys Ser Leu Glu Leu Lys Glu Ser Ala Pro Thr Gly Thr Thr Thr Ala
        140                 145                 150

gcg ccg act gaa aaa gtg ccc ccc agt ggc ccg cga tca ggt gaa gtt    531
Ala Pro Thr Glu Lys Val Pro Pro Ser Gly Pro Arg Ser Gly Glu Val
    155                 160                 165

cag cgt act cgt aaa gag caa aat gac gtg cag caa acc gca gag atg    579
Gln Arg Thr Arg Lys Glu Gln Asn Asp Val Gln Gln Thr Ala Glu Met
170                 175                 180                 185

ttg gct gag gaa att ctt gag gct ggg ctt aag aag gac gat gga gaa    627
Leu Ala Glu Glu Ile Leu Glu Ala Gly Leu Lys Lys Asp Asp Gly Glu
                190                 195                 200

gga cgg gga acg cca gaa gct gaa gtc aat taa gaaaatcact aaacgtcaag 680

Gly Arg Gly Thr Pro Glu Ala Glu Val Asn
            205                 210

ttctttatga ctgctgtaca ccaccacccc cctggactgc ttaagacagc taacaagcgt 740

tggatttcaa tatcctactt aaggtatgtg gggcggatgt cgtgtcacgg tgtgtatggc 800

gttaaaaaac ggcacacggc attaaatgca gtgcaagtat gaattgtgcg caggttgtgt 860

gtgacatttt tcggatgtcc tgggctttgt gtgcgtgcgt gggctgcgaa gagattagat 920

ttatttcttg cgattgcgat gcgtagtttg ttgcatcgtt atggtcatga aaaaagtcta 980

acgacacaca taaacgatgg agcaaattaa aaaaaaaaaa aaaaaaaaaa a          1031

<210> 5
<211> 211
<212> PRT
<213> Neospora caninum

<400> 5
Met Phe Thr Gly Lys Arg Trp Ile Leu Val Val Ala Val Gly Ala Leu
  1               5                  10                  15
Val Gly Ala Ser Val Lys Ala Ala Asp Phe Ser Gly Arg Gly Thr Val
             20                  25                  30
Asn Gly Gln Pro Val Gly Ser Gly Tyr Ser Gly Tyr Pro Arg Gly Asp
         35                  40                  45
Asp Val Arg Glu Ser Met Ala Ala Pro Glu Asp Leu Pro Gly Glu Arg
     50                  55                  60
Gln Pro Glu Thr Pro Thr Ala Glu Ala Val Lys Gln Ala Ala Ala Lys
 65                  70                  75                  80
Ala Tyr Arg Leu Leu Lys Gln Phe Thr Ala Lys Val Gly Gln Glu Thr
                 85                  90                  95
Glu Asn Ala Tyr Tyr His Val Lys Lys Ala Thr Met Lys Gly Phe Asp
            100                 105                 110
Val Ala Lys Asp Gln Ser Tyr Lys Gly Tyr Leu Ala Val Arg Lys Ala
        115                 120                 125
Thr Ala Lys Gly Leu Gln Ser Ala Gly Lys Ser Leu Glu Leu Lys Glu
    130                 135                 140
Ser Ala Pro Thr Gly Thr Thr Thr Ala Ala Pro Thr Glu Lys Val Pro
145                 150                 155                 160
Pro Ser Gly Pro Arg Ser Gly Glu Val Gln Arg Thr Arg Lys Glu Gln
                165                 170                 175
Asn Asp Val Gln Gln Thr Ala Glu Met Leu Ala Glu Glu Ile Leu Glu
            180                 185                 190
Ala Gly Leu Lys Lys Asp Asp Gly Glu Gly Arg Gly Thr Pro Glu Ala
        195                 200                 205

Glu Val Asn
    210

<210> 6
<211> 1263
<212> DNA
<213> Neospora caninum

<220>
<221> CDS
<222> (130)..(1089)

<400> 6
tctgcgtgca gccttccgtt gttctcgctt gtatcacagg tgcctttgtc gtacataaac 60

attgtttcga ttgtagtcta gtcacaccgc actcgtttca tcactggcgc ttttgtttat 120

tcatcgaat atg ttt cct cgg gca gtg aga cgc gcc gtc tcg gtg ggt gtg 171
          Met Phe Pro Arg Ala Val Arg Arg Ala Val Ser Val Gly Val
          1               5                   10

ttc gcc gcg ccc gca ctg gtg gcg ttc ttt gac tgt gga act atg gca 219
Phe Ala Ala Pro Ala Leu Val Ala Phe Phe Asp Cys Gly Thr Met Ala
15                  20                  25                  30

tca gaa aaa tca cct cta ctt gtc aat caa gtt gtc acc tgt gac aac 267
Ser Glu Lys Ser Pro Leu Leu Val Asn Gln Val Val Thr Cys Asp Asn
                35                  40                  45

gaa gag aaa tca tca gtt gcc gtc cta cta tca ccg aag ctg aac cac 315
Glu Glu Lys Ser Ser Val Ala Val Leu Leu Ser Pro Lys Leu Asn His
            50                  55                  60

atc acg ctc aag tgc cct gac aat tcg acc gcc gtg ccc gct gct ctt 363
Ile Thr Leu Lys Cys Pro Asp Asn Ser Thr Ala Val Pro Ala Ala Leu
        65                  70                  75

ggt tat cca aca aac agg acc gtc tgc ccg gcg gag tcc gga ggt caa 411
Gly Tyr Pro Thr Asn Arg Thr Val Cys Pro Ala Glu Ser Gly Gly Gln
    80                  85                  90

act tgt aca ggc aag gag ata ccg ttg gaa agc ctg ctt ccc ggg gca 459
Thr Cys Thr Gly Lys Glu Ile Pro Leu Glu Ser Leu Leu Pro Gly Ala
95                  100                 105                 110

aac gat agc tgg tgg tca ggt gtt gat atc aag act ggc gtt aag ctc 507

Asn Asp Ser Trp Trp Ser Gly Val Asp Ile Lys Thr Gly Val Lys Leu
                115                 120                 125

aca att cct gaa gcg agc ttc ccc aca aca tcc aag tcg ttc gac gtc 555
Thr Ile Pro Glu Ala Ser Phe Pro Thr Thr Ser Lys Ser Phe Asp Val
            130                 135                 140

ggc tgc gtc agc agt gat gcc agc aag agt tgt atg gtc aca gtc aca 603
Gly Cys Val Ser Ser Asp Ala Ser Lys Ser Cys Met Val Thr Val Thr
        145                 150                 155

gtg cca ccc aga gcc tca tcg ctt gtc aac ggt gtc gca atg tgc tct 651
Val Pro Pro Arg Ala Ser Ser Leu Val Asn Gly Val Ala Met Cys Ser
    160                 165                 170

tac ggt gca aac gaa act ctc ggc cct atc aca ttg tcc gag ggc gga 699
Tyr Gly Ala Asn Glu Thr Leu Gly Pro Ile Thr Leu Ser Glu Gly Gly
175                 180                 185                 190

tct tct acg atg acc ctc gtt tgc ggc acg gat ggg aag cca gtt cct 747
Ser Ser Thr Met Thr Leu Val Cys Gly Thr Asp Gly Lys Pro Val Pro
                195                 200                 205

cct gat cct aag cag gtt tgt tct ggg acg acc gtc aag gat tgt aaa 795
Pro Asp Pro Lys Gln Val Cys Ser Gly Thr Thr Val Lys Asp Cys Lys
            210                 215                 220

gca aaa ccg ttc act gat gtt ttc cca aaa ttc agt gct gat tgg tgg 843
Ala Lys Pro Phe Thr Asp Val Phe Pro Lys Phe Ser Ala Asp Trp Trp
        225                 230                 235

cag gga aaa ccc gac act aag gat ggt gca aaa cta acg atc aag aaa 891
Gln Gly Lys Pro Asp Thr Lys Asp Gly Ala Lys Leu Thr Ile Lys Lys
    240                 245                 250

ggt gca ttt cct cca aag gag gaa aag ttt act ctt ggg tgc aag agc 939
Gly Ala Phe Pro Pro Lys Glu Glu Lys Phe Thr Leu Gly Cys Lys Ser
255                 260                 265                 270

gta tcg agt ccg gag gtt tac tgt act gtg cag gtg gag gca gag cgc 987
Val Ser Ser Pro Glu Val Tyr Cys Thr Val Gln Val Glu Ala Glu Arg
                275                 280                 285

gcg agt gca ggg atc aag tcg tcg gct gaa aat gtt ggt cgc gtt tcc 1035
Ala Ser Ala Gly Ile Lys Ser Ser Ala Glu Asn Val Gly Arg Val Ser
            290                 295                 300

ctt ttc gct gta aca att gga ctc gta ggc tcg ata gcg gct ggc gtc 1083

Leu Phe Ala Val Thr Ile Gly Leu Val Gly Ser Ile Ala Ala Gly Val
        305                 310                 315

gcg tga gtgacaatcg ttctgctcgc cattcataaa aataatgcaa gacatgttcg    1139
Ala
    320

cgttcgtcat gtgtgtcttt atcataaaac aacatttact gattacttgt ggtggtttgc 1199

atatgtacaa tcccaaaaac tgctctactg taaagacgtt tagagtaaaa aaaaaaaaaa 1259

aaaa                                                              1263

<210> 7
<211> 319
<212> PRT
<213> Neospora caninum

<400> 7
Met Phe Pro Arg Ala Val Arg Arg Ala Val Ser Val Gly Val Phe Ala
  1               5                  10                  15
Ala Pro Ala Leu Val Ala Phe Phe Asp Cys Gly Thr Met Ala Ser Glu
             20                  25                  30
Lys Ser Pro Leu Leu Val Asn Gln Val Val Thr Cys Asp Asn Glu Glu
         35                  40                  45
Lys Ser Ser Val Ala Val Leu Leu Ser Pro Lys Leu Asn His Ile Thr
     50                  55                  60
Leu Lys Cys Pro Asp Asn Ser Thr Ala Val Pro Ala Ala Leu Gly Tyr
 65                  70                  75                  80
Pro Thr Asn Arg Thr Val Cys Pro Ala Glu Ser Gly Gly Gln Thr Cys
                 85                  90                  95
Thr Gly Lys Glu Ile Pro Leu Glu Ser Leu Leu Pro Gly Ala Asn Asp
            100                 105                 110
Ser Trp Trp Ser Gly Val Asp Ile Lys Thr Gly Val Lys Leu Thr Ile
        115                 120                 125
Pro Glu Ala Ser Phe Pro Thr Thr Ser Lys Ser Phe Asp Val Gly Cys
    130                 135                 140
Val Ser Ser Asp Ala Ser Lys Ser Cys Met Val Thr Val Thr Val Pro
145                 150                 155                 160
Pro Arg Ala Ser Ser Leu Val Asn Gly Val Ala Met Cys Ser Tyr Gly
                165                 170                 175
Ala Asn Glu Thr Leu Gly Pro Ile Thr Leu Ser Glu Gly Gly Ser Ser
            180                 185                 190
Thr Met Thr Leu Val Cys Gly Thr Asp Gly Lys Pro Val Pro Pro Asp
        195                 200                 205
Pro Lys Gln Val Cys Ser Gly Thr Thr Val Lys Asp Cys Lys Ala Lys
    210                 215                 220

Pro Phe Thr Asp Val Phe Pro Lys Phe Ser Ala Asp Trp Trp Gln Gly
225                 230                 235                 240
Lys Pro Asp Thr Lys Asp Gly Ala Lys Leu Thr Ile Lys Lys Gly Ala
                245                 250                 255
Phe Pro Pro Lys Glu Glu Lys Phe Thr Leu Gly Cys Lys Ser Val Ser
            260                 265                 270
Ser Pro Glu Val Tyr Cys Thr Val Gln Val Glu Ala Glu Arg Ala Ser
        275                 280                 285
Ala Gly Ile Lys Ser Ser Ala Glu Asn Val Gly Arg Val Ser Leu Phe
    290                 295                 300
Ala Val Thr Ile Gly Leu Val Gly Ser Ile Ala Ala Gly Val Ala
305                 310                 315

<210> 8
<211> 2069
<212> DNA
<213> Neospora caninum

<220>
<221> CDS
<222> (138)..(1520)

<400> 8
tcaatccttt cccgtgctaa cttgtaaaat cgctgctttc gttggttggt tttgtttcac 60

gtggctgtta agaggtcgac gcagctgttt aacccgtgcc cctggttctc caggtgatct 120

gcatcggatt tgcaaag atg ggc cag tcg gtg gtt ttc gtc atg ctt ttg     170
                   Met Gly Gln Ser Val Val Phe Val Met Leu Leu
                   1               5                   10

tcg gta ata ttt acc gct ggg gca aaa aca tac gga gaa gcg tcg caa     218
Ser Val Ile Phe Thr Ala Gly Ala Lys Thr Tyr Gly Glu Ala Ser Gln
            15                  20                  25

cca tcg gcc tca gca cgt tcg tta cag ggg gcc ctc gat aca tgg tgc     266
Pro Ser Ala Ser Ala Arg Ser Leu Gln Gly Ala Leu Asp Thr Trp Cys
        30                  35                  40

cag gag gtt ttt aaa aaa ctg tgc gat gac gga tat tca aaa atg tgt     314
Gln Glu Val Phe Lys Lys Leu Cys Asp Asp Gly Tyr Ser Lys Met Cys
    45                  50                  55

att cca gcc aac cag gta gtt gca cga caa ggc ctg ggt aga aaa gac     362
Ile Pro Ala Asn Gln Val Val Ala Arg Gln Gly Leu Gly Arg Lys Asp
60                  65                  70                  75

caa caa aag ctc gta tgg cgg tgc tac gat tca gcg gcg ttt ctg gcc 410
Gln Gln Lys Leu Val Trp Arg Cys Tyr Asp Ser Ala Ala Phe Leu Ala
                80                  85                  90

gaa ggc gac gaa aac aat gtc ctc agc tgc gtg gac gac tgt ggc gtt 458
Glu Gly Asp Glu Asn Asn Val Leu Ser Cys Val Asp Asp Cys Gly Val
            95                  100                 105

tcg ata ccg tgt cct ggc gga gtt gat agg gat aat agt acc cac gct 506
Ser Ile Pro Cys Pro Gly Gly Val Asp Arg Asp Asn Ser Thr His Ala
        110                 115                 120

acg cga cat gat gag ctt tcc caa tta atc aag gaa gga gta gtg cgc 554
Thr Arg His Asp Glu Leu Ser Gln Leu Ile Lys Glu Gly Val Val Arg
    125                 130                 135

tat tgc agt ggt ttc caa gcg gct gcc aac agc tac tgc aac aaa cga 602
Tyr Cys Ser Gly Phe Gln Ala Ala Ala Asn Ser Tyr Cys Asn Lys Arg
140                 145                 150                 155

tat cct ggg act gtt gcg agg aag tcg aag ggc ttc gga cac aag gaa 650
Tyr Pro Gly Thr Val Ala Arg Lys Ser Lys Gly Phe Gly His Lys Glu
                160                 165                 170

cca gtt aaa tgg aga tgt tac aag cca gag agc tta tta ttt tcg gtt 698
Pro Val Lys Trp Arg Cys Tyr Lys Pro Glu Ser Leu Leu Phe Ser Val
            175                 180                 185

ttt tct gag tgc gtg agt aac tgc gga aca acc tgg tcc tgc cct gga 746
Phe Ser Glu Cys Val Ser Asn Cys Gly Thr Thr Trp Ser Cys Pro Gly
        190                 195                 200

gga cga tta ggg aca gcg aca aat cta gac aaa aag cat ttc aca gat 794
Gly Arg Leu Gly Thr Ala Thr Asn Leu Asp Lys Lys His Phe Thr Asp
    205                 210                 215

gag tcc ggg att ctc cag gca ctc acc tct gtg ccg aaa gca tgt cca 842
Glu Ser Gly Ile Leu Gln Ala Leu Thr Ser Val Pro Lys Ala Cys Pro
220                 225                 230                 235

gta ggc ctt gtt tgc ctc ccg agg gat cag aat ccc ccg gcg tgt tta 890
Val Gly Leu Val Cys Leu Pro Arg Asp Gln Asn Pro Pro Ala Cys Leu
                240                 245                 250

gat gat aac ggc aac gtc cca gaa gag gag gga ggg cag ccc gta caa 938
Asp Asp Asn Gly Asn Val Pro Glu Glu Glu Gly Gly Gln Pro Val Gln
            255                 260                 265

ccg cgt gac acg aag ttg ccc gtt gat gat tcg gaa ccg acc gat gaa    986
Pro Arg Asp Thr Lys Leu Pro Val Asp Asp Ser Glu Pro Thr Asp Glu
        270                 275                 280

agt gaa act aca cct ggt gga ggt gat gat cag ccg agc cca aaa gag    1034
Ser Glu Thr Thr Pro Gly Gly Gly Asp Asp Gln Pro Ser Pro Lys Glu
    285                 290                 295

gac ggg gac aca gac tca cct gat gaa ggt gac cag tcc ggg ggt tca    1082
Asp Gly Asp Thr Asp Ser Pro Asp Glu Gly Asp Gln Ser Gly Gly Ser
300                 305                 310                 315

gag tgg tac aaa cag att ccg gaa atc cgt gtc atc ggt gac agc ctg    1130
Glu Trp Tyr Lys Gln Ile Pro Glu Ile Arg Val Ile Gly Asp Ser Leu
                320                 325                 330

caa gca atg ctc cac gct ggg cag cag ctg atg gtc acc tat agc tct    1178
Gln Ala Met Leu His Ala Gly Gln Gln Leu Met Val Thr Tyr Ser Ser
            335                 340                 345

ccc caa ctc cat gtt agt gtg gga tca tgt cac aaa ctc acg gtg aat    1226
Pro Gln Leu His Val Ser Val Gly Ser Cys His Lys Leu Thr Val Asn
        350                 355                 360

ttc tcc gat tat tat ttg tct ttt gac acc acc tca aag tcg ggg tcc    1274
Phe Ser Asp Tyr Tyr Leu Ser Phe Asp Thr Thr Ser Lys Ser Gly Ser
    365                 370                 375

gac gaa gtg gaa ctg gac gat gca gcg gga agc gga gag ctc acg ata    1322
Asp Glu Val Glu Leu Asp Asp Ala Ala Gly Ser Gly Glu Leu Thr Ile
380                 385                 390                 395

gga ctg gga agc agc ggc cgt gtg act gtt gtc ttc cag tat gcc aca    1370
Gly Leu Gly Ser Ser Gly Arg Val Thr Val Val Phe Gln Tyr Ala Thr
                400                 405                 410

aac ggt ggg gga aac aga tat gtt gct tac acc gtc gga gat tct gga    1418
Asn Gly Gly Gly Asn Arg Tyr Val Ala Tyr Thr Val Gly Asp Ser Gly
            415                 420                 425

tgc aaa aca att gaa gct gtt ctc ctt cac ggc ctg aat cct gga gcg    1466
Cys Lys Thr Ile Glu Ala Val Leu Leu His Gly Leu Asn Pro Gly Ala
        430                 435                 440

aag ctc gtt agg aat acg ata ggc gat aat tct ccg ggt gaa tct gaa    1514
Lys Leu Val Arg Asn Thr Ile Gly Asp Asn Ser Pro Gly Glu Ser Glu
    445                 450                 455

ttg taa cgactctttg tgttagtagt agccctccct atacagaatg ggagtgtatt 1570
Leu
460

acattttgtg atcaagggaa gaggagcgat cactacactt gatcacgcgt cgaggtcatt 1630

cgtgcggggc tgcagcttta tggtttgatc acgcaagaaa agaagcgcaa cacctgcaag 1690

tcgggcatgc gcgagggtcc catccttagt tttttttagt tttttttttg ccttcccgtc 1750

cgtccatatt tctcgggtct gtattttcta gcctgagatt ctagcctaga tccaatgcag 1810

tatgtcgcct gaagtcatgt taagtggtca gatgtttctg tctcagtgaa gaaaactgtg 1870

ttatggtgca ttctgtccga ttttatacgt aattcgtcgt acgttccatt gagttacgtg 1930

aggatgcgaa cgcagcaagt gatgtacgac aagttcgtag catggtgaca ctgtagaata 1990

caagtgtatt ttacagtcag gcggccggct actacacatt caagctgagt gacgtcgctt 2050

caaaaaaaaa aaaaaaaaa 2069

<210> 9
<211> 460
<212> PRT
<213> Neospora caninum

<400> 9
Met Gly Gln Ser Val Val Phe Val Met Leu Leu Ser Val Ile Phe Thr
1               5                   10                  15
Ala Gly Ala Lys Thr Tyr Gly Glu Ala Ser Gln Pro Ser Ala Ser Ala
            20                  25                  30
Arg Ser Leu Gln Gly Ala Leu Asp Thr Trp Cys Gln Glu Val Phe Lys
        35                  40                  45
Lys Leu Cys Asp Asp Gly Tyr Ser Lys Met Cys Ile Pro Ala Asn Gln
    50                  55                  60
Val Val Ala Arg Gln Gly Leu Gly Arg Lys Asp Gln Gln Lys Leu Val
65                  70                  75                  80
Trp Arg Cys Tyr Asp Ser Ala Ala Phe Leu Ala Glu Gly Asp Glu Asn
                85                  90                  95
Asn Val Leu Ser Cys Val Asp Asp Cys Gly Val Ser Ile Pro Cys Pro
            100                 105                 110
Gly Gly Val Asp Arg Asp Asn Ser Thr His Ala Thr Arg His Asp Glu
        115                 120                 125
Leu Ser Gln Leu Ile Lys Glu Gly Val Val Arg Tyr Cys Ser Gly Phe
    130                 135                 140

Gln Ala Ala Ala Asn Ser Tyr Cys Asn Lys Arg Tyr Pro Gly Thr Val
145                 150                 155                 160
Ala Arg Lys Ser Lys Gly Phe Gly His Lys Glu Pro Val Lys Trp Arg
                165                 170                 175
Cys Tyr Lys Pro Glu Ser Leu Leu Phe Ser Val Phe Ser Glu Cys Val
            180                 185                 190
Ser Asn Cys Gly Thr Thr Trp Ser Cys Pro Gly Gly Arg Leu Gly Thr
        195                 200                 205
Ala Thr Asn Leu Asp Lys Lys His Phe Thr Asp Glu Ser Gly Ile Leu
    210                 215                 220
Gln Ala Leu Thr Ser Val Pro Lys Ala Cys Pro Val Gly Leu Val Cys
225                 230                 235                 240
Leu Pro Arg Asp Gln Asn Pro Pro Ala Cys Leu Asp Asp Asn Gly Asn
                245                 250                 255
Val Pro Glu Glu Glu Gly Gly Gln Pro Val Gln Pro Arg Asp Thr Lys
            260                 265                 270
Leu Pro Val Asp Asp Ser Glu Pro Thr Asp Glu Ser Glu Thr Thr Pro
        275                 280                 285
Gly Gly Gly Asp Asp Gln Pro Ser Pro Lys Glu Asp Gly Asp Thr Asp
    290                 295                 300
Ser Pro Asp Glu Gly Asp Gln Ser Gly Gly Ser Glu Trp Tyr Lys Gln
305                 310                 315                 320
Ile Pro Glu Ile Arg Val Ile Gly Asp Ser Leu Gln Ala Met Leu His
                325                 330                 335
Ala Gly Gln Gln Leu Met Val Thr Tyr Ser Ser Pro Gln Leu His Val
            340                 345                 350
Ser Val Gly Ser Cys His Lys Leu Thr Val Asn Phe Ser Asp Tyr Tyr
        355                 360                 365
Leu Ser Phe Asp Thr Thr Ser Lys Ser Gly Ser Asp Glu Val Glu Leu
    370                 375                 380
Asp Asp Ala Ala Gly Ser Gly Glu Leu Thr Ile Gly Leu Gly Ser Ser
385                 390                 395                 400
Gly Arg Val Thr Val Val Phe Gln Tyr Ala Thr Asn Gly Gly Gly Asn
                405                 410                 415
Arg Tyr Val Ala Tyr Thr Val Gly Asp Ser Gly Cys Lys Thr Ile Glu
            420                 425                 430
Ala Val Leu Leu His Gly Leu Asn Pro Gly Ala Lys Leu Val Arg Asn
        435                 440                 445
Thr Ile Gly Asp Asn Ser Pro Gly Glu Ser Glu Leu
    450                 455                 460

<210> 10
<211> 2278
<212> DNA
<213> Neospora caninum

<400> 10
atgggccagt cggtggtttt cgtcatgctt ttgtcggtaa tatttaccgc tggggcaaaa 60
acatacggag aaggtaagtc tccagctggt ttgtttgctt tgcaacaccc cccacctgga 120
gcgtctcgca actgtagatt gaagaaacta gtggacccgg ttgctggttc ttcaggtacc 180
gtagtacatt cattggcaac agtgtagtcc ttttcgcata gtagcaaggc gtcgaactgt 240
ttttagtccg gatacaatcg gacgttctgc attgcgtgcg aactgctgtg aggacacctt 300
ctgatgcacg gaactgattt tctggatttg tcgggtgttt gcagcgtcgc aaccatcggc 360
ctcagcacgt tcgttacagg gggccctcga tacatggtgc caggaggttt ttaaaaaact 420
gtgcgatgac ggatattcaa aaatgtgtat tccagccaac caggtagttg cacgacaagg 480
cctgggtaga aaagaccaac aaaagctcgt atggcggtgc tacgattcag cggcgtttct 540
ggccgaaggc gacgaaaaca atgtcctcag ctgcgtggac gactgtggcg tttcgatacc 600
gtgtcctggc ggagttgata gggataatag tacccacgct acgcgacatg atgagctttc 660
ccaattaatc aaggaaggag tagtgcgcta ttgcagtggt ttccaagcgg ctgccaacag 720
ctactgcaac aaacgatatc ctgggactgt tgcgaggaag tcgaagggct tcggacacaa 780
ggaaccagtt aaatggagat gttacaagcc agtaaggagg agctggctag attgcattag 840
tctgccctca ccacatcgtc agcgatcgct tcttgtgggg gataggagac atgatcctgg 900
gtcgcggaag agatgagcct ggtcctcgtc cgtgttagtg gcagcaaatt aaccccacgg 960
aggtggcagg gattatttag catagcgtat gtacgttttc ggtggagggc aggagcacga 1020
gataactgta gagatccacg gcctctgtgc ctttccagtt atgttcacac agttttacac 1080
tagctgatag cattcacata cgttttacga agttcccgac aaacaccaag aggaaagtgg 1140
gggaaatgtt agatttgagg tgcgtactgt tgttgatgtg ttttaggaga gcttattatt 1200
ttcggttttt tctgagtgcg tgagtaactg cggaacaacc tggtcctgcc ctggaggacg 1260
attaggtgag tttaagattc aggaatagca gaaatagtgc cacgaggtgc agcttcagcc 1320
tgtaacgctg cttcttcatc actcgtatcc tggacacccc gagaaaggca tcggattgtt 1380
tttcaggatt taccaaacaa acaatgatgc gagtcgagca gttattctgg gatttttttt 1440
ctagaatgtg taagccagtt tcaatcgttg gctcatccgg catctttttc ctgttggcgc 1500
tcggttactt gcagggacag cgacaaatct agacaaaaag catttcacag atgagtccgg 1560
gattctccag gcactcacct ctgtgccgaa agcatgtcca gtaggccttg tttgcctccc 1620
gagggatcag aatcccccgg cgtgtttaga tgataacggc aacgtcccag aagaggaggg 1680
agggcagccc gtacaaccgc gtgacacgaa gttgcccgtt gatgattcgg aaccgaccga 1740
tgaaagtgaa actacacctg gtggaggtga tgatcagccg agcccaaaag aggacgggga 1800
cacagactca cctgatgaag gtgaccagtc cgggggttca gagtggtaca aacagattcc 1860
ggaaatccgt gtcatcggtg acagcctgca agcaatgctc cacgctgggc agcagctgat 1920
ggtcacctat agctctcccc aactccatgt tagtgtggga tcatgtcaca aactcacagt 1980
gaatttctcc gattattatt tgtcttttga caccacctca aagtcggggt ccgacgaagt 2040
ggaactggac gatgcagcgg gaagcggaga gctcacgata ggactgggaa gcagcggccg 2100
tgtgactgtt gtcttccagt atgccacaaa cggtggggga aacagatatg ttgcttacac 2160
cgtcggagat tctggatgca aaacaattga agctgttctc cttcacggcc tgaatcctgg 2220
agcgaagctc gttaggaata cgataggcga taattctccg ggtgaatctg aattgtaa 2278

<210> 11
<211> 4242
<212> DNA
<213> Neospora caninum

<400> 11
cgggaattcg attccagccg agttcgtgtt cagacgtgta cgccacccac ggtacaattt 60

attcagggga gagaaaaaga agtcgcggca aaaacataaa gctcttcggc agttcaacga 120
tgaaactgca aattaatgaa atgatttaaa ctaatcccag cgcagaacgg agttgcaata 180
accttccgac agagacgcac cggaggacct gggaaccgat ccgaaatgag acgaggacag 240
caacttgaga cggctcattg cgtcgcaact gtgtttgcgt aaccgctgcg agcagttccg 300
ccagcccatg agtgggatgt tcgtgtgctt gggatactag gcggttcacc tgaaaggcgg 360
tcgtcccgct cttaagagcc gccgcgcaac agccgaagcg ctagggaccg ttggtctacc 420
atcgccccat cgctcatgaa ctctagtggg gagagcgtca gctccgcaac aggtcgtgta 480
tctggaggct acagtgagcg tcgcatttca ctcgccagta ctagcagcct tggcctttgg 540
tagcgttgcg atggcctatg ccagtgcgag cgcgctaaac tactggcagt agacacacca 600
tctggtgagc tctycctatg tctaaaacgt gaagatgagc gcgtgtgtgc ggatgacaga 660
ggtatcaaga catctgtcag gtagaaattt tcttttaaca gttgaacaat cgtttcgtga 720
ctctctggtg gtctgtctgt gtactagatg tactctttcc caagccgctc gaggaacata 780
cgtgaagcac ggtggtactt cctgtagcaa aacatagcag gtaaaggtga tgtggttcga 840
aactgcagtg tgtactgtac tttggggggt ggcgcacggt tgggcacgcc catttgctag 900
gggttcgggg aagggaaggg tgtgtggttg acggtttatc atcagagaaa ctggagtggg 960
gacaagatta taatacgtca agctgcaagc cgctgtagtt ggagaaagct gtttttgagg 1020
ggcatacttg tttgcgcgga tgatgtacgt atttcaccta aacatgttga aatacgctcg 1080
ctgagaaacg gaggcaaaat tcaggaggaa tggggaaggg tacccgtatg gtgagcacgc 1140
gttgcaaaag cacaagtagg agaacacgcg acatagaaca actcggcggc catgtgtgta 1200
aacggcatta acggatgttt ccacccttac acactctcga tgcgtgggac aatggagtcg 1260
atgaaaacga tgcatggttt ggttgcatga ctgtgcgcag cacaatggcg ttcggacgag 1320
gcaagagggg actgcacgct gcagtcatct taggcttctt tgtcctcctc gccacatcat 1380
ctgtaggatt gggccaaagg tgagtcaaag catgacgtgt tttgctacgt gtaggaacag 1440
cacgttgcgg ttcgaccact tgctcagtag ggtcatgcaa cactttgtgc tgattcaact 1500
ggtgtgcagg gtgcctcgct acccaagtgt ggagtcactc gaagaaagag ttgccgaggc 1560
tctagggcgc cgtagctccg cagcggccag tactcttcca gggagtgaca cgaacatgat 1620
atcagatggt cgcgcaggca gggatgaacc aacagcgagc ccagagcatc attccgtgga 1680
cgctccgacc acgtctgggg aaggcgaggc agatgctggg aaagtaacgc tgaggaacga 1740
tgagggcctt gagggtaata tctcagccga ccatgttcta catccccctc ctgacagtga 1800
acacgagggt ttgcaggaac cgggcacgac gcatcaggag gcgcaagaac cagacgcgag 1860
tgaagcaatg gactcttccg cgctaccact ttccacgtcg ggtaccacat cctaacgaag 1920
tcggttcaac accaggaaca gcgctgcctg ccccgatttt tagcattcca gagctctcac 1980
cggaggaagt tgtctacgtt ttacgggttc agggatcagg cgatttcgaa attagtttcc 2040
aagtaggccg ggtggtgagg cagttggaag ccatcaagag agcatacagg gaggctcacg 2100
ggaagctaga agctgaggag ctggagtcgg aaaggggacc gacggtttcg actcggacga 2160
aactagttga ctttatcaaa gaaaaccaga gacggctgag ggcggcgttt cagaaggtta 2220
agattcagca aaagttggag gagatcgagg aactgttgca gctgtcacac gcactaaaat 2280
ctctaggtgc ccgcctgaga ccctgccaaa aaagtaattc cccaatggag gaagagattt 2340
gtcgtaagac gaaagctttg ggcgaaatgg ttgcccagaa agcggaggat cttcgtcagc 2400
atgcgtcaac tgtctcggct ctgctaggtc gcgaagctgt tgagagacag ttgcggcgtg 2460
tcgacagtga acaaccctat gaacaaacag acgccggggt tgcagccaga gcagaggaat 2520
ttcggaaggc actggagaaa gcagcttccg gtgcgagaca attcgtgggg accacagcgg 2580
acgaaatagt ggaggaagtg aaggaggatg ctcagtacct gcgtgatggt gcgaaagaag 2640
tgttgacgaa gagccagcgc gcgctagtag acgcgtttca ggcgatccaa agggctctac 2700
tggaggcgaa ggcaaaggag ctcgtagatg ccgcatcaaa ggaagctgaa gacgctcgta 2760
agatcttagc ggaacagcca gcgtgattcg ccgaggacga agttggtaat gcacggtgaa 2820
tgagggttgg tcatcccaat ccccagcttg atagcgtcac gtgggttttt cgccggggaa 2880
acgatcatta gggaggtgat gtatcgcagt aaacatgggc atatcagcac cagttttttt 2940

acatgtgagg gatgggatcc agtgtaggtg taagggacag ctgtctttca aatttgggct 3000
tcggttgccg ctcccgttct ttcagcatat gtacaggtat gtacagtgaa taagtgcgtg 3060
ggccaatgtg ctctcatcaa tcatgtacag aacatatgtt ttggtcatat ctatgcagcg 3120
cctgcatgag cccatgccgc tcgtgtttta cgaagccaga tgcggtgccg ccctgtccca 3180
gctacacatg ctgtgcacgg ggaacaacgg ccatgttgga aaagtcactg ttttataaat 3240
gattgacaac taatgaaaaa gcactcaagc gggaaatgtt tcatgcggtc caaagagcag 3300
gggggaaagt cactgtttta taaatgattg tgacaactat ctaatgaaaa agcactcaag 3360
cgggaaatgt ttcatgcggt ccaaagagta gggggcgggc gtggtactga tgattaccgc 3420
gtaacaatga ccacgccggc gcagatgtcg cagtgctgta gcgtttgatg ttcttttgta 3480
tggcggaagg gtgacaaggc aaacggcgag agtcgactta cagactcacc accgggcaac 3540
catcggttcc caggtcaata agctggacta ttgtcagcag atgcgatgat aacgcgtgcc 3600
atacataaag agcgtacccg tgctagttaa agatgcgcac gcggttctgt tggcagaggt 3660
cggaggcttg cctcatggag caaccgaggg ggcgcagttc tgtcttcgtg tcttccgttt 3720
gtgtgtttga gaacgaacag atacggcgta tgtgcttgcc ttggtcacag ggagctcacc 3780
acaaagcccg tgtagtcggg ggagtactgc tggacacagt ggcgagaata cgcgtgatca 3840
atgccggcaa tagagaaatc ggcatgaaat tgtgtagcgg atggcgttct gtatgtcgta 3900
caagcgaccc tggatcgtgt gtacccccct acgggcgggc tgccctgtga aggcaatata 3960
aaatgtaatc caatgattcg ttttcatgtt acaccagata ttcttaggac gatggtactg 4020
accatactag catctgagta gtagtctctc ggtgttcggt ggccaatcta cgactctagc 4080
aatgggttcc ctctctaccc taggttccgt agtgtgggca catcacatga tgactgtcga 4140
tccagaaatt gatacacgtg catgcttctc agctatgaca attatgattg ctattcctac 4200
agcagccaac cggatccgaa ttcttcgccc tatagtgaag tc 4242

<210> 12
<211> 1892
<212> DNA
<213> Neospora caninum

<220>
<221> CDS
<222> (122)..(1381)

<400> 12
gaacaatcgt ttcgtgactc tctggtggtc tgtctgtgta ctagatgtac tctttcccaa 60

gccgctcgag gaacatacgt gaagcacggt ggtacttcct gtagcaaaac atagcagcac 120

a atg gcg ttc gga cga ggc aag agg gga ctg cac gct gca gtc atc tta 169
  Met Ala Phe Gly Arg Gly Lys Arg Gly Leu His Ala Ala Val Ile Leu
  1               5                   10                  15

ggc ttc ttt gtc ctc ctc gcc aca tca tct gta gga ttg ggc caa agg 217
Gly Phe Phe Val Leu Leu Ala Thr Ser Ser Val Gly Leu Gly Gln Arg
            20                  25                  30

gtg cct cgc tac cca agt gtg gag tca ctc gaa gaa aga gtt gcc gag 265
Val Pro Arg Tyr Pro Ser Val Glu Ser Leu Glu Glu Arg Val Ala Glu

        35                  40                  45

gct cta ggg cgc cgt agc tcc gca gcg gcc agt act ctt cca ggg agt 313
Ala Leu Gly Arg Arg Ser Ser Ala Ala Ala Ser Thr Leu Pro Gly Ser
    50                  55                  60

gac acg aac atg ata tca gat ggt cgc gca ggc agg gat gaa cca aca 361
Asp Thr Asn Met Ile Ser Asp Gly Arg Ala Gly Arg Asp Glu Pro Thr
65                  70                  75                  80

gcg agc cca gag cat cat tcc gtg gac gct ccg acc acg tct ggg gaa 409
Ala Ser Pro Glu His His Ser Val Asp Ala Pro Thr Thr Ser Gly Glu
                85                  90                  95

ggc gag gca gat gct ggg aaa gta acg ctg agg aac gat gag ggc ctt 457
Gly Glu Ala Asp Ala Gly Lys Val Thr Leu Arg Asn Asp Glu Gly Leu
            100                 105                 110

gag ggt aat atc tca gcc gac cat gtt cta cat ccc cct cct gac agt 505
Glu Gly Asn Ile Ser Ala Asp His Val Leu His Pro Pro Pro Asp Ser
        115                 120                 125

gaa cac gag gtc ggt tca aca cca gga aca gcg ctg cct gcc ccg att 553
Glu His Glu Val Gly Ser Thr Pro Gly Thr Ala Leu Pro Ala Pro Ile
    130                 135                 140

ttt agc att cca gag ctc tca ccg gag gaa gtt gtc tac gtt tta cgg 601
Phe Ser Ile Pro Glu Leu Ser Pro Glu Glu Val Val Tyr Val Leu Arg
145                 150                 155                 160

gtt cag gga tca ggc gat ttc gaa att agt ttc caa gta ggc cgg gtg 649
Val Gln Gly Ser Gly Asp Phe Glu Ile Ser Phe Gln Val Gly Arg Val
                165                 170                 175

gtg agg cag ttg gaa gcc atc aag aga gca tac agg gag gct cac ggg 697
Val Arg Gln Leu Glu Ala Ile Lys Arg Ala Tyr Arg Glu Ala His Gly
            180                 185                 190

aag cta gaa gct gag gag ctg gag tcg gaa agg gga ccg acg gtt tcg 745
Lys Leu Glu Ala Glu Glu Leu Glu Ser Glu Arg Gly Pro Thr Val Ser
        195                 200                 205

act cgg acg aaa cta gtt gac ttt atc aaa gaa aac cag aga cgg ctg 793
Thr Arg Thr Lys Leu Val Asp Phe Ile Lys Glu Asn Gln Arg Arg Leu
    210                 215                 220

agg gcg gcg ttt cag aag gtt aag att cag caa aag ttg gag gag atc 841
Arg Ala Ala Phe Gln Lys Val Lys Ile Gln Gln Lys Leu Glu Glu Ile

225                 230                 235                 240

gag gaa ctg ttg cag ctg tca cac gca cta aaa tct cta ggt gcc cgc     889
Glu Glu Leu Leu Gln Leu Ser His Ala Leu Lys Ser Leu Gly Ala Arg
                245                 250                 255

ctg aga ccc tgc caa aaa agt aat tcc cca atg gag gaa gag att tgt     937
Leu Arg Pro Cys Gln Lys Ser Asn Ser Pro Met Glu Glu Glu Ile Cys
            260                 265                 270

cgt aag acg aaa gct ttg ggc gaa atg gtt gcc cag aaa gcg gag gat     985
Arg Lys Thr Lys Ala Leu Gly Glu Met Val Ala Gln Lys Ala Glu Asp
        275                 280                 285

ctt cgt cag cat gcg tca act gtc tcg gct ctg cta ggt cgc gaa gct     1033
Leu Arg Gln His Ala Ser Thr Val Ser Ala Leu Leu Gly Arg Glu Ala
    290                 295                 300

gtt gag aga cag ttg cgg cgt gtc gac agt gaa caa ccc tat gaa caa     1081
Val Glu Arg Gln Leu Arg Arg Val Asp Ser Glu Gln Pro Tyr Glu Gln
305                 310                 315                 320

aca gac gcc ggg gtt gca gcc aga gca gag gaa ttt cgg aag gca ctg     1129
Thr Asp Ala Gly Val Ala Ala Arg Ala Glu Glu Phe Arg Lys Ala Leu
                325                 330                 335

gag aaa gca gct tcc ggt gcg aga caa ttc gtg ggg acc aca gcg gac     1177
Glu Lys Ala Ala Ser Gly Ala Arg Gln Phe Val Gly Thr Thr Ala Asp
            340                 345                 350

gaa ata gtg gag gaa gtg aag gag gat gct cag tac ctg cgt gat ggt     1225
Glu Ile Val Glu Glu Val Lys Glu Asp Ala Gln Tyr Leu Arg Asp Gly
        355                 360                 365

gcg aaa gaa gtg ttg acg aag agc cag cgc gcg cta gta gac gcg ttt     1273
Ala Lys Glu Val Leu Thr Lys Ser Gln Arg Ala Leu Val Asp Ala Phe
    370                 375                 380

cag gcg atc caa agg gct cta ctg gag gcg aag gca aag gag ctc gta     1321
Gln Ala Ile Gln Arg Ala Leu Leu Glu Ala Lys Ala Lys Glu Leu Val
385                 390                 395                 400

gat gcc gca tca aag gaa gct gaa gac gct cgt aag atc tta gcg gaa     1369
Asp Ala Ala Ser Lys Glu Ala Glu Asp Ala Arg Lys Ile Leu Ala Glu
                405                 410                 415

cag cca gcg tga ttcgccgagg acgaagttgg taatgcacgg tgaatgaggg     1421
Gln Pro Ala

420

ttggtcatcc caatccccag cttgatagcg tcacgtgggt ttttcgccgg ggaaacgatc 1481

attagggagg tgatgtatcg cagtaaacat gggcatatca gcaccagttt ttttacatgt 1541

gagggatggg atccagtgta ggtgtaaggg acagctgtct ttcaaatttg ggcttcggtt 1601

gccgctcccg ttctttcagc atatgtacag gtatgtacag tgaataagtg cgtgggccaa 1661

tgtgctctca tcaatcatgt acagaacata tgttttggtc atatctatgc agcgcctgca 1721

tgagcccatg ccgctcgtgt tttacgaagc cagatgcggt gccgccctgt cccagctaca 1781

catgctgtgc acggggaaca acggccatgt tggaaaagtc actgttttat aaatgattga 1841

caactaatga aaaagcactc aagcgggaaa tgtttcatgc ggtccaaaga g 1892

<210> 13
<211> 419
<212> PRT
<213> Neospora caninum

<400> 13
Met Ala Phe Gly Arg Gly Lys Arg Gly Leu His Ala Ala Val Ile Leu
1               5                   10                  15
Gly Phe Phe Val Leu Leu Ala Thr Ser Ser Val Gly Leu Gly Gln Arg
            20                  25                  30
Val Pro Arg Tyr Pro Ser Val Glu Ser Leu Glu Glu Arg Val Ala Glu
        35                  40                  45
Ala Leu Gly Arg Arg Ser Ser Ala Ala Ala Ser Thr Leu Pro Gly Ser
    50                  55                  60
Asp Thr Asn Met Ile Ser Asp Gly Arg Ala Gly Arg Asp Glu Pro Thr
65                  70                  75                  80
Ala Ser Pro Glu His His Ser Val Asp Ala Pro Thr Thr Ser Gly Glu
                85                  90                  95
Gly Glu Ala Asp Ala Gly Lys Val Thr Leu Arg Asn Asp Glu Gly Leu
            100                 105                 110
Glu Gly Asn Ile Ser Ala Asp His Val Leu His Pro Pro Pro Asp Ser
        115                 120                 125
Glu His Glu Val Gly Ser Thr Pro Gly Thr Ala Leu Pro Ala Pro Ile
    130                 135                 140
Phe Ser Ile Pro Glu Leu Ser Pro Glu Glu Val Val Tyr Val Leu Arg
145                 150                 155                 160
Val Gln Gly Ser Gly Asp Phe Glu Ile Ser Phe Gln Val Gly Arg Val
                165                 170                 175
Val Arg Gln Leu Glu Ala Ile Lys Arg Ala Tyr Arg Glu Ala His Gly

            180                 185                 190
Lys Leu Glu Ala Glu Glu Leu Glu Ser Glu Arg Gly Pro Thr Val Ser
        195                 200                 205
Thr Arg Thr Lys Leu Val Asp Phe Ile Lys Glu Asn Gln Arg Arg Leu
    210                 215                 220
Arg Ala Ala Phe Gln Lys Val Lys Ile Gln Gln Lys Leu Glu Glu Ile
225                 230                 235                 240
Glu Glu Leu Leu Gln Leu Ser His Ala Leu Lys Ser Leu Gly Ala Arg
                245                 250                 255
Leu Arg Pro Cys Gln Lys Ser Asn Ser Pro Met Glu Glu Glu Ile Cys
            260                 265                 270
Arg Lys Thr Lys Ala Leu Gly Glu Met Val Ala Gln Lys Ala Glu Asp
        275                 280                 285
Leu Arg Gln His Ala Ser Thr Val Ser Ala Leu Leu Gly Arg Glu Ala
    290                 295                 300
Val Glu Arg Gln Leu Arg Arg Val Asp Ser Glu Gln Pro Tyr Glu Gln
305                 310                 315                 320
Thr Asp Ala Gly Val Ala Ala Arg Ala Glu Glu Phe Arg Lys Ala Leu
                325                 330                 335
Glu Lys Ala Ala Ser Gly Ala Arg Gln Phe Val Gly Thr Thr Ala Asp
            340                 345                 350
Glu Ile Val Glu Glu Val Lys Glu Asp Ala Gln Tyr Leu Arg Asp Gly
        355                 360                 365
Ala Lys Glu Val Leu Thr Lys Ser Gln Arg Ala Leu Val Asp Ala Phe
    370                 375                 380
Gln Ala Ile Gln Arg Ala Leu Leu Glu Ala Lys Ala Lys Glu Leu Val
385                 390                 395                 400
Asp Ala Ala Ser Lys Glu Ala Glu Asp Ala Arg Lys Ile Leu Ala Glu
                405                 410                 415
Gln Pro Ala

<210> 14
<211> 20
<212> DNA
<213> Neospora caninum

<400> 14
aattaaccct cactaaaggg 20

<210> 15
<211> 22
<212> DNA
<213> Neospora caninum

<400> 15

gtaatacgac tcactatagg gc 22

<210> 16
<211> 20
<212> DNA
<213> Neospora caninum

<400> 16
gccgcgactt ctttttctct 20

<210> 17
<211> 20
<212> DNA
<213> Neospora caninum

<400> 17
ctcgatcgcc tcctttactg 20

<210> 18
<211> 20
<212> DNA
<213> Neospora caninum

<400> 18
tgctagtact ggcgagtgaa 20

<210> 19
<211> 20
<212> DNA
<213> Neospora caninum

<400> 19
caggtttgcc acacattttt 20

<210> 20
<211> 21
<212> DNA
<213> Neospora caninum

<400> 20
atgtttcctc ctcgggcagt g 21

<210> 21
<211> 23
<212> DNA
<213> Neospora caninum

<400> 21
tcacgcgacg ccagccgcta tcg                                                23

<210> 22
<211> 20
<212> DNA
<213> Neospora caninum

<400> 22
gccctgacaa ttcgaccgcc                                                    20

<210> 23
<211> 21
<212> DNA
<213> Neospora caninum

<400> 23
cccacaacat ccaagtcgtt c                                                  21

<210> 24
<211> 20
<212> DNA
<213> Neospora caninum

<400> 24
gttttgcacc atccttagtg                                                    20

<210> 25
<211> 19
<212> DNA
<213> Neospora caninum

<400> 25
gagagtttgc tttgcaccg                                                     19

<210> 26
<211> 21
<212> DNA

<213> Neospora caninum

<400> 26
ccagccgagt tcgtgttcag a                                                21

<210> 27
<211> 24
<212> DNA
<213> Neospora caninum

<400> 27
caacgtggat ccgattcaag cttc                                             24

<210> 28
<211> 20
<212> DNA
<213> Neospora caninum

<400> 28
aaagctcttc ggcagttcaa                                                  20

<210> 29
<211> 18
<212> DNA
<213> Neospora caninum

<400> 29
ccgcgctacc actttcca                                                    18

<210> 30
<211> 18
<212> DNA
<213> Neospora caninum

<400> 30
gtaatacgac tcactata                                                    18

<210> 31
<211> 19
<212> DNA
<213> Neospora caninum

<400> 31

ccgcaacgtg ctgttccta 19

<210> 32
<211> 18
<212> DNA
<213> Neospora caninum

<400> 32
catcagagaa actggagt 18

<210> 33
<211> 24
<212> DNA
<213> Neospora caninum

<400> 33
ggccaagctt gctagtactg gcga 24

<210> 34
<211> 31
<212> DNA
<213> Neospora caninum

<400> 34
atccaatgca tcttgctgaa tgccttaaaa g 31

## Description

### 1. FIELD OF THE INVENTION

The present invention is in the field of animal health, and is directed to vaccine compositions and diagnostics for disease. More particularly, the present invention relates to polynucleotide molecules comprising nucleotide sequences encoding GRA1, GRA2, SAG1, MIC1, and MAG1 proteins from *Neospora,* which polynucleotide molecules and proteins are useful in the production of vaccines against neosporosis, and as diagnostic reagents.

### 2. BACKGROUND OF THE INVENTION

*Neospora* is a pathogenic protozoan parasite of animals that has been recognized as a major cause of abortion, neonatal death, congenital infection, and encephalitic disease in mammals. Dubey and Lindsay, 1996, Vet. Parasitol. 67:1-59; Dubey and Lindsay, 1993, Parasitology Today, 9:452-458. *Neospora caninum* infects dogs, and congenitally infects pups, often leading to paralysis. Tachyzoites of *N. caninum* have been isolated from naturally infected pups. Lindsay and Dubey, 1989, J. Parasitol. 75:163-165. Neospora is a major cause of abortion in dairy and beef cattle. Cases of Neospora-related disease, *i.e.,* neosporosis, have also been reported in goats, sheep and horses.

Although *N*. *caninum* is superficially similar to the pathogen, *Toxoplasma gondii*, *N. caninum* and *T. gondii* have been distinguished from each other both antigenically and ultrastructurally. Dubey and Lindsay, 1993, above. In addition, *Neospora*-like protozoan parasites isolated from the brains of aborted bovine fetuses and continuously cultured *in vitro* were shown to be antigenically and ultrastructurally distinct from both *T. gondii* and *Hammondia hammondi,* and were most similar to *N. caninum*. Conrad *et al*., 1993, Parasitology 106:239-249. Furthermore, analysis of nuclear small subunit ribosomal RNA genes revealed no nucleotide differences between strains of *Neospora* isolated from cattle and dogs, but showed consistent differences between Neospora and *T. gondii.* Marsh *et al*., 1995, J. Parasitol. 81:530-535.

The etiologic role of a bovine isolate of *Neospora* in bovine abortion and congenital disease has been confirmed. Barr *et al*., 1994, J. Vet Diag. Invest. 6:207-215. A rodent model of central nervous system neosporosis has been developed using inbred BALB/c mice infected with *N. caninum.* Lindsay et *al.,* 1995, J. Parasitol. 81:313-315. In addition, models to study transplacental transmission of *N. caninum* in pregnant outbred and inbred mice have been described by Cole *et al*., 1995, J. Parasitol. 81:730-732, and by Long *et al*., 1996, J. Parasitol. 82:608-611, respectively. An experimental *N. caninum* pygmy goat model that closely resembles naturally acquired *Neospora*-induced cattle abortion has been demonstrated. Lindsay *et al*., 1995, Am. J. Vet. Res. 56:1176-1180. An experimental *N. caninum* sheep model that closely resembles naturally acquired *Neospora*-induced cattle abortion has also been demonstrated. Buxton *et al*., 1997, J. Comp. Path. 117:1-16.

In *T. gondii,* electron dense granules comprising an excretory-secretory group of antigens are present in the cytoplasm of tachyzoites. These antigens have been designated as GRA proteins. The GRA1 protein of *T*. *gondii* has been reported to have a molecular weight ranging from about 22-27 kDa, and the GRA2 protein of *T. gondii* has been reported to have a molecular weight of about 28 kDa. Sam-Yellowe. 1996, Parasitol. Today 12:308-315. Similar electron dense granules are present in the cytoplasm of N. *caninum* tachyzoites (Bjerkas *et al*., 1994, Clin. Diag. Lab. Immunol. 1:214-221; Hemphill *et al*., 1998, Intl. J. Parasitol. 28:429-438).

*T. gondii* cells are also known to comprise a group of major surface antigens that have been designated as SAG. The SAG1 protein of *T. gondii* is reported to have a molecular weight of about 30 kDa. Kasper *et al*., 1983, J. Immunol. 130:2407-2412. Monoclonal antibodies directed against *T. gondii* SAG1 protein significantly blocked the ability of *T. gondii* tachyzoites to invade bovine kidney cells under tissue culture conditions. Grimwood and Smith, 1996, Intl. J. Parasitol. 26: 169-173. Because *T. gondii* SAG1 appears to play a role in the invasion process, it has been hypothesized that SAG1 may be necessary to support the virulence phenotype. Windeck and Gross, 1996, Parasitol. Res. 82:715-719. Consistent with this hypothesis is the observation that mice immunized with *T. gondii* SAG1 protein and then challenged with *T. gondii* had reduced toxoplasma cyst formation in their brains than did control mice. Debard *et al*., 1996, Infect. Immun., 64:2158-2166. *T. gondii* SAG1 may be functionally related to a similar molecule in *N. caninum* designated as NC-p36 described by Hemphill *et al*., 1997, Parasitol. 115:371-380.

Micronemes are intracelluar organelles located at the apical end of tachyzoites of both *T. gondii* and *Neospora,* and may play a role in host cell recognition and attachment to the host cell surface during invasion. Formaux *et al*., 1996, Curr. Top. Microbiol. Immunol. 219:55-58. At least 4 different microneme-associated (MIC) proteins have been identified in *T. gondii.* The MIC1 protein of *T. gondii* is about 60 kDa, binds to the surface of host cells, and has been reported to have partial homology to thrombospondin-related adhesive protein (TRAP) from *Plasmodium falciparum* which binds to human hepatocytes. Robson *et al*. 1995 EMBO J. 14:3883-3894.

The conversion of parasites from tachyzoites to bradyzoites is critical for chronic infection and persistence of *T*. *gondii.* A gene expressing an immunodominant, bradyzoite-specific 65 kD antigen, designated as *MAG1*, has been identified in *T. gondii*. Parmley *et al*., 1994, Mol. Biochem. Parasitol. 66:283-296. *MAG1* has been reported to be specifically expressed in bradyzoite cysts, but not in the tachyzoite stage. This specificity of expression may indicate the involvement of *MAG1* in the conversion between tachyzoite and bradyzoite stages of the life cycle of the parasite. Bohne et al., 1996, Curr. Topics Microbiol. Immunol. 219:81-91.

Identification in *Neospora* of protein homologs of *T*. *gondii* GRA1, GRA2, SAG1, MIC1, and MAG1 proteins, and the nucleotide sequence of polynucleotide molecules encoding said *Neospora* proteins, will serve to facilitate the development of vaccines against neosporosis, as well as diagnostic reagents.

### 3. SUMMARY OF THE INVENTION

The present invention provides an isolated polynucleotide molecule comprising a nucleotide sequence encoding the GRA1 protein from *N. caninum.* In a preferred embodiment, the GRA1 protein has the amino acid sequence of SEQ ID NO:2. In a further preferred embodiment, the isolated GRA1-encoding polynucleotide molecule of the present invention comprises a nucleotide sequence selected from the group consisting of the nucleotide sequence of SEQ ID NO:1 from about nt 205 to about nt 777, the nucleotide sequence of the open reading frame (ORF) of the GRA1 gene, which is presented in SEQ ID NO:3 from about nt 605 to about nt 1304, and the nucleotide sequence of the GRA1-encoding ORF of plasmid pRC77 (ATCC 209685). In a non-limiting embodiment. the isolated GRA1-encoding polynucleotide molecule of the present invention comprises a nucleotide sequence selected from the group consisting of the nucleotide sequence of SEQ ID NO:1 and SEQ ID NO:3. The present invention further provides an isolated polynucleotide molecule having a nucleotide sequence that is homologous to the nucleotide sequence of a GRA1-encoding polynucleotide molecule of the present invention. The present invention further provides an isolated polynucleotide molecule comprising a nucleotide sequence that encodes a polypeptide that is homologous to the GRA1 protein of *N. caninum.* The present invention further provides a polynucleotide molecule consisting of a nucleotide sequence that is a substantial portion of any of the aforementioned *GRA1*-related polynucleotide molecules.

The present invention further provides an isolated polynucleotide molecule comprising a nucleotide sequence encoding the GRA2 protein from *N. caninum.* In a preferred embodiment, the GRA2 protein has the amino acid sequence of SEQ ID NO:5. In a further preferred embodiment, the isolated GRA2-encoding polynucleotide molecule of the present invention comprises a nucleotide sequence selected from the group consisting of the nucleotide sequence of the ORF of SEQ ID NO:4, which is from about nt 25 to about nt 660, and the nucleotide sequence of the GRA2-encoding ORF of plasmid pRC5 (ATCC 209686). In a non-limiting embodiment, the isolated GRA2-encoding polynucleotide molecule of the present invention comprises the nucleotide sequence of SEQ ID NO:4. The present invention further provides an isolated polynucleotide molecule having a nucleotide sequence that is homologous to the nucleotide sequence of a GRA2-encoding polynucleotide molecule of the present invention. The present invention further provides an isolated polynucleotide molecule comprising a nucleotide sequence that encodes a polypeptide that is homologous to the GRA2 protein of *N. caninum*. The present invention further provides a polynucleotide molecule consisting of a nucleotide sequence that is a substantial portion of any of the aforementioned GRA2-related polynucleotide molecules.

The present invention further provides an isolated polynucleotide molecule comprising a nucleotide sequence encoding the SAG1 protein from *N. caninum.* In a preferred embodiment, the SAG1 protein has the amino acid sequence of SEQ ID NO:7. In a further preferred embodiment, the isolated SAG1-encoding polynucleotide molecule of the present invention comprises a nucleotide sequence selected from the group consisting of the nucleotide sequence of the ORF of SEQ ID NO:6, which is from about nt 130 to about nt 1089, and the nucleotide sequence of the SAG1-encoding ORF of plasmid pRC102 (ATCC 209687). In a non-limiting embodiment, the isolated SAG1-encoding polynucleotide molecule of the present invention comprises the nucleotide sequence of SEQ ID NO:6. The present invention further provides an isolated polynucleotide molecule having a nucleotide sequence that is homologous to the nucleotide sequence of a SAG1-encoding polynucleotide molecule of the present invention. The present invention further provides an isolated polynucleotide molecule comprising a nucleotide sequence that encodes a polypeptide that is homologous to the SAG1 protein of *N. caninum.* The present invention further provides a polynucleotide molecule consisting of a nucleotide sequence that is a substantial portion of any of the aforementioned *SAG1*-related polynucleotide molecules.

The present invention further provides an isolated polynucleotide molecule comprising a nucleotide sequence encoding the MIC1 protein from *N. caninum.* In a preferred embodiment, the MIC1 protein has the amino acid sequence of SEQ ID NO:9. In a further preferred embodiment, the isolated MIC1-encoding polynucleotide molecule of the present invention comprises a nucleotide sequence selected from the group consisting of the nucleotide sequence of the ORF of SEQ ID NO:8 from about nt 138 to about nt 1520, the nucleotide sequence of the ORF of the *MIC1* gene, which is presented as SEQ ID NO:10, and the nucleotide sequence of the MIC1-encoding ORF of plasmid pRC340 (ATCC 209688). In a non-limiting embodiment, the isolated MIC1-encoding polynucleotide molecule of the present invention comprises a nucleotide sequence selected from the group consisting of the nucleotide sequence of SEQ ID NO:8, and the nucleotide sequence of SEQ ID NO:10. The present invention further provides an isolated polynucleotide molecule having a nucleotide sequence that is homologous to the nucleotide sequence of a MIC1-encoding polynucleotide molecule of the present invention. The present invention further provides an isolated polynucleotide molecule comprising a nucleotide sequence that encodes a polypeptide that is homologous to the MIC1 protein of *N. caninum.* The present invention further provides a polynucleotide molecule consisting of a nucleotide sequence that is a substantial portion of any of the aforementioned *MIC1*-related polynucleotide molecules.

The present invention further provides an isolated polynucleotide molecule comprising a nucleotide sequence encoding the MAG1 protein from *N. caninum.* The MAG1 protein has a putative amino acid sequence shown in SEQ ID NO:13. In a preferred embodiment, the isolated MAG1-encoding polynucleotide molecule of the present invention comprises a nucleotide sequence selected from the group consisting of the nucleotide sequence presented in SEQ ID NO:11 from about nt 1305 to about nt 2786. a cDNA molecule prepared therefrom, such as a cDNA molecule having the ORF of SEQ ID NO:12 from about nt 122 to about nt 1381, and the nucleotide sequence of the MAGI-encoding ORF present in plasmid bd304 (ATCC 203413). The present invention further provides a polynucleotide molecule having a nucleotide sequence of any ORF present in SEQ ID NO:11. In a non-limiting embodiment, the isolated MAGI-encoding polynucleotide molecule of the present invention comprises a nucleotide sequence selected from the group consisting of SEQ ID NO:11 and SEQ ID NO:12. The present invention further provides an isolated polynucleotide molecule having a nucleotide sequence that is homologous to the nucleotide sequence of a MAGI-encoding polynucleotide molecule of the present invention. The present invention further provides an isolated polynucleotide molecule comprising a nucleotide sequence that encodes a potypeptide that is homologous to the MAG1 protein of *N. caninum.* The present invention further provides a polynucleotide molecule consisting of a nucleotide sequence that is a substantial portion of any of the aforementioned *MAG1*-related polynucleotide molecules.

The present invention further provides a polynucleotide molecule comprising the nucleotide sequence of the promoters of the *N. caninum GRA1* and *MAG1* genes, which is presented in SEQ ID NO:11 from about nt 127 to about nt 703, and includes its complementary sequence.

The present invention further provides oligonucleotide molecules that hybridize to any of the polynucleotide molecules of the present invention, or that hybridize to a polynucleotide molecule having a nucleotide sequence that is the complement of any of the polynucleotide molecules of the present invention.

The present invention further provides compositions and methods for cloning and expressing any of the polynucleotide molecules of the present invention, including recombinant cloning vectors, recombinant expression vectors, transformed host cells comprising any of said vectors, and novel strains or cell lines derived therefrom. More particularly, the present invention provides a recombinant vector comprising a polynucleotide molecule having a nucleotide sequence encoding the GRA1, GRA2, SAG1, MIC1 or MAG1 protein of *N. caninum.* In specific, though non-limiting, embodiments, the present invention provides plasmid pRC77 (ATCC 209685) encoding GRA1; plasmid pRC5 (ATCC 209686) encoding GRA2; plasmid pRC102 (ATCC 209687) encoding SAG1; plasmid pRC340 (ATCC 209688) encoding MIC1; and plasmid bd304 (ATCC 203413) comprising the *MAG1* gene sequence and the *MAG1*/*GRA1* bidirectional promoter region.

The present invention further provides a substantially purified or isolated *N caninum* polypeptide selected from the group consisting of GRA1, GRA2, SAG1, MIC1 and MAG1 proteins. In a preferred embodiment, the *N. caninum* GRA1 protein has the amino acid sequence of SEQ ID NO:2. In another preferred embodiment, the *N. caninum* GRA2 protein has the amino acid sequence of SEQ ID NO:5. In another preferred embodiment, the *N. caninum* SAG1 protein has the amino acid sequence of SEQ ID NO:7. In another preferred embodiment, the *N. caninum* MIC1 protein has the amino acid sequence of SEQ ID NO:9. In another preferred embodiment, the *N. caninum* MAG1 protein has the amino acid sequence of SEQ ID NO:13. The present invention further provides substantially purified or isolated polypeptides that are homologous to any of the aforementioned *N. caninum* proteins. The present invention further provides polypeptides which are fusion proteins comprising any of the aforementioned polypeptides fused to a carrier or fusion partner, as known in the art. The present invention further provides polypeptides consisting of a substantial portion of any of the aforementioned polypeptides. The polypeptides of the present invention are useful both in vaccine compositions to protect mammals against neosporosis and as diagnostic reagents.

The present invention further provides a method of preparing any of the aforementioned polypeptides, comprising culturing host cells transformed with a recombinant expression vector, said vector comprising a polynucleotide molecule comprising a nucleotide sequence encoding any of the aforementioned potypeptides, wherein the nucleotide sequence is in operative association with one or more regulatory elements, under conditions conducive to the expression of the polypeptide, and recovering the expressed polypeptide from the cell culture.

The present invention further provides antibodies specifically directed against a *N.caninum* GRA1, GRA2, SAG1, MIC1 or MAG1 protein.

The present invention further provides genetic constructs for use in mutating a *Neospora GRA1*, *GRA2, SAG1*, *MIC1* or *MAG1* gene to produce modified *Neospora* cells. Such modified Neospora cells are useful in vaccine compositions to protect mammals against neosporosis. In a preferred though non-limiting embodiment, a genetic construct of the present invention comprises a polynucleotide molecule comprising a nucleotide sequence that is otherwise the same as a nucleotide sequence encoding a GRA1, GRA2, SAG1, MIC1 or MAG1 protein from *N. caninum,* or a substantial portion thereof, but that further comprises one or more mutations *i.e*., one or more nucleotide deletions, insertions and/or substitutions, that can serve to mutate the gene. Once transformed into cells of *Neospora*. the polynucleotide molecule of the genetic construct is specifically targeted, e.g., by homologous recombination, to the particular *Neospora* gene, and either deletes or replaces the gene or a portion thereof, or inserts into the gene. As a result of this recombination event, the *Neospora* gene is mutated. The resulting mutated gene is preferably partially or fully disabled in that it encodes either a partially cefective or fully defective protein, or fails to encode a protein. The present invention further provides *Neospora* cells which have been modified by one or more of said gene mutations and methods of preparing modified *Neospora* cells using a genetic construct of the present invention.

The present invention further provides a vaccine against neosporosis, comprising an immunologically effective amount of a polypeptide of the present invention, or an immunologically effective amount of a polynucleotide molecule of the present invention, or an immunologically effective amount of modified *Neospora* cells of the present invention; and a veterinarily acceptable carrier. In a preferred embodiment, the vaccine of the present invention comprises modified live cells of *N. caninum* that express a *GRA1*⁻, *GRA2*⁻, *SAG1*⁻, *MIC1*⁻ or *MAG1*⁻ phenotype, or a combination of said phenotypes. In a non-limiting embodiment, the vaccine is a combination vaccine for protecting a mammal against neosporosis and, optionally. one or more other diseases or pathological conditions that can afflict the mammal, which combination vaccine comprises an immunologically effective amount of a first component comprising a polypeptide, polynucleotide molecule, or modified *Neospora* cells of the present invention, an immunologically effective amount of a second component that is different from the first component, and that is capable of inducing, or contributing to the induction of, a protective response against a disease or pathological condition that can afflict the mammal; and a veterinarily acceptable carrier.

The present invention further provides a method of preparing a vaccine against neosporosis, comprising combining an immunologically effective amount of a *N. caninum* polypeptice of the present invention, or an immunologically effective amount of a polynucleotide molecule of the present invention, or an immunologically effective amount of modified *Neospora* cells of the present invention, with a veterinarily acceptable carrier, in a form suitable for administration to a mammal.

The present invention further provides a method of vaccinating a mammal against neosporosis, comprising administering to the mammal an immunologically effective amount of a vaccine of the present invention.

The present invention further provides a kit for vaccinating a mammal against neosporosis, comprising a first container having an immunologically effective amount of a polypeptide of the present invention, or an immunologically effective amount of a polynucleotide molecule of the present invention, or an immunologically effective amount of modified *Neospora* cells of the present invention; and a second container having a veterinarily acceptable carrier or diluent.

### 4. DETAILED DESCRIPTION OF THE INVENTION

### 4.1. Polynucleotide Molecules

An isolated polynucleotide molecule of the present invention can have a nucleotide sequence derived from any species or strain of *Neospora,* but is preferably from a pathogenic species of *Neospora* such as *N. caninum.* A non-limiting example of a strain of *N. caninum* from which a polynucleotide molecule of the present invention can be isolated or derived is strain NC-1, which is available in host MARC-145 monkey kidney cells under Accession No. CRL-12231 from the American Type Culture Collection (ATCC), located at 12301 Parklawn Drive. Rockville, MD 20852, USA. Strain NC-1 is also described in Dubey *et al*., 1988, J. Am. Vet. Med. Assoc. 193:1259-63, which publication is incorporated herein by reference. Altematively, pathogenic strains or species of *Neospora* for use in practicing the present invention can be isolated from organs, tissues or body fluids of infected animals using standard isolation techniques such as those described in the publications reviewed above.

As used herein, the terms "polynucleotide molecule." "polynucleotide sequence," "coding sequence," "open-reading frame (ORF)," and the like, are intended to refer to both DNA and RNA molecules, which can either be single-stranded or double-stranded, and that can include one or more prokaryotic sequences, cDNA sequences, genomic DNA sequences including exons and introns, and chemically synthesized DNA and RNA sequences, and both sense and corresponding anti-sense strands. As used herein, the term "ORF" refers to the minimal nucleotide sequence required to encode a particular Neospora protein, *i.e*., either a GRA1, GRA2, SAG1, MIC1 or MAG1 protein, without any intervening termination codons.

Production and manipulation of the polynucleotide molecules and oligonucleotide molecules disclosed herein are within the skill in the art and can be carried out according to recombinant techniques described, among other places, in Maniatis *et al*., 1989, Molecular Cloning, A Laboratory Manual, Cold Spring Harbor Laboratory Press, Cold Spring Harbor, NY; Ausubel et al., 1989, Current Protocols In Molecular Biology, Greene Publishing Associates & Wiley Interscience, NY; Sambrook *et al*., 1989, Molecular Cloning: A Laboratory Manual, 2d ed., Cold Spring Harbor Laboratory Press, Cold Spring Harbor, NY; Innis *et al*. (eds), 1995, PCR Strategies, Academic Press, Inc., San Diego; and Erlich (ed), 1992, PCR Technology, Oxford University Press, New York, all of which are incorporated herein by reference.

### 4.1.1. GRA1-Related Polynucleotide Molecules

References herein below to the nucleotide sequences shown in SEQ ID NOS:1 and 3. and to substantial portions thereof, are intended to also refer to the corresponding nucleotide sequences and substantial portions thereof, respectively, as present in plasmid pRC77 (ATCC 209685), unless otherwise indicated. In addition, references herein below to the amino acid sequences shown in SEQ ID NO:2, and to substantial portions and peptide fragments thereof. are intended to also refer to the corresponding amino acid sequences, and substantial portions and peptide fragments thereof, respectively, encoded by the corresponding GRA1-encoding nucleotide sequence present in plasmid pRC77 (ATCC 209685). unless otherwise indicated.

The present invention provides an isolated polynucleotide molecule comprising a nucleotide sequence encoding the GRA1 protein from *N. caninum.* In a preferred embodiment, the GRA1 protein has the amino acid sequence of SEQ ID NO:2. In a further preferred embodiment, the isolated GRA1-encoding polynucleotide molecule of the present invention comprises a nucleotide sequence selected from the group consisting of the nucleotide sequence of SEQ ID NO:1 from about nt 205 to about nt 777, the nucleotide sequence of the open reading frame (ORF) of the *GRA1* gene, which is presented in SEQ ID NO:3 from about nt 605 to about nt 1304, and the nucleotide sequence of the GRA1-encoding ORF of plasmid pRC77 (ATCC 209685). In a non-limiting embodiment, the isolated GRA1-encoding polynucleotide molecule of the present invention comprises a nucleotide sequence selected from the group consisting of the nucleotide sequence of SEQ ID NO:1 and SEQ ID NO:3. The GRA1 gene presented in SEQ ID NO:3 comprises an ORF from nt 605 to nt 855 and from nt 983 to nt 1304 with an intervening intron extending from nt 856 to nt 982. In addition, putative promoter motifs have been identified within 150 bp 5' of the mRNA start site that are similar to those found in *T. gondii* GRA genes (see Section 5.3, below).

The present invention further provides an isolated polynucleotide molecule having a nucleotide sequence that is homologous to the nucleotide sequence of a GRA1-encoding polynucleotide molecule of the present invention. The term "homologous" when used to refer to a GRA1-related polynucleotide molecule means a polynucleotide molecule having a nucleotide sequence: (a) that encodes the same protein as one of the aforementioned GRA1-encoding polynucleotide molecules of the present invention, but that includes one or more silent changes to the nucleotide sequence according to the degeneracy of the genetic code; or (b) that hybridizes to the complement of a polynucleotide molecule having a nucleotide sequence that encodes the amino acid sequence of the *N. caninum* GRA1 protein, under moderately stringent conditions, *i.e.,* hybridization to filter-bound DNA in 0.5 M NaHPO₄. 7% sodium dodecyl sulfate (SDS), 1 mM EDTA at 65°C. and washing in 0.2xSSC/0.1% SDS at 42°C (see Ausubel *et al*. (eds.), 1989. Current Protocols in Molecular Biology, Vol. I, Green Publishing Associates, Inc., and John Wiley & Sons, Inc., New York, at p. 2.10.3), and that is useful in practicing the present invention. In a preferred embodiment, the homologous polynucleotide molecule hybridizes to the complement of a polynucleotide molecule having a nucleotide sequence that encodes the amino acid sequence of the *N. caninum* GRA1 protein under highly stringent conditions, *i.e*., hybridization to filter-bound DNA in 0.5 M NaHPO₄, 7% SDS, 1 mM EDTA at 65°C, and washing in 0.1xSSC/0.1% SDS at 68°C (Ausubel *et al*., 1989, above), and is useful in practicing the present invention. In a more preferred embodiment, the homologous polynucleotide molecule hybridizes under highly stringent conditions to the complement of a polynucleotide molecule consisting of a nucleotide sequence selected from the group consisting of the ORF of SEQ ID NO:1, which is from about nt 205 to about nt 777, and the ORF of the *GRA1* gene, which is presented in SEQ ID NO:3 from about nt 605 to about nt 1304, and which is useful in practicing the present invention.

As used herein, a polynucleotide molecule is "useful in practicing the present invention" where the polynucleotide molecule can be used to amplify a *Neospora*-specific polynucleotide molecule using standard amplification techniques, or as a diagnostic reagent to detect the presence of a *Neospora*-specific polynucleotide in a fluid or tissue sample from a *Neospora*-infected animal.

Polynucleotide molecules of the present invention having nucleotide sequences that are homologous to the nucleotide sequence of a GRA1-encoding polynucleotide molecule of the present invention do not include polynucleotide molecules having the native nucleotide sequence of *T. gondii* encoding a *T*. *gondii* GRA protein, and further have no more than about 90%, and preferably no more than about 80%, sequence identity to such a *T. gondii* polynucleotide molecule, wherein sequence identity is determined by use of the BLASTN algorithm (GenBank, National Center for Biotechnology Information).

The present invention further provides an isolated polynucleotide molecule comprising a nucleotide sequence that encodes a polypeptide that is homologous to the *N. caninum* GRA1 protein. As used herein to refer to polypeptides that are homologous to the *N. caninum* GRA1 protein, the term "homologous" refers to a polypeptide otherwise having the amino acid sequence of the *N. caninum* GRA1 protein, but in which one or more amino acid residues have been conservatively substituted with a different amino acid residue, where the resulting polypeptide is useful in practicing the present invention. Conservative amino acid substitutions are well-known in the art. Rules for making such substitutions include those described by Dayhof. M.D., 1978, Nat. Biomed. Res. Found., Washington. D.C., Vcl. 5, Sup 3, among others. More specifically, conservative amino acid substitutions are those that generally take place within a family of amino acids that are related in acidity, polarity, or bulkiness of their side chains. Genetically encoded amino acids are generally divided into four groups. (1) acidic = aspartate, glutamate: (2) basic = lysine, arginine, histidine; (3) non-polar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan and tyrosine are also jointly classified as aromatic amino acids. One or more replacements within any particular group, *e.g*., of a leucine with an isoleucine or valine, or of an aspartate with a glutamate, or of a threonine with a serine, or of any other amino acid residue with a structurally related amino acid residue, *e.g*., an amino acid residue with similar acidity, polarity, bulkiness of side chain, or with similarity in some combination thereof, will generally have an insignificant effect on the function or immunogenicity of the polypeptide.

As used herein, a polypeptide is "useful in practicing the present invention" where the polypeptide can be used as a diagnostic reagent to detect the presence of *Neospora*-specific antibodies in a blood or serum sample from an animal that is currently infected, or that has been infected, with *Neospora.*

The present invention further provides a polynucleotide molecule consisting of a substantial portion of any of the aforementioned *Neospora GRA1*-related polynucleotide molecules of the present invention. As used herein, a "substantial portion" of a *GRA1*-related polynucleotide molecule means a polynucleotide molecule consisting of less than the complete nucleotide sequence of the *GRA1*-related polynucleotide molecule, but comprising at least about 5%, and more preferably at least about 10%, of the nucleotide sequence of the *GRA1*-related polynucleotide molecule, and that is useful in practicing the present invention, as usefulness is defined above for polynucleotide molecules.

In addition to the nucleotide sequences of any of the aforementioned *GRA1*-related polynucleotide molecules, polynucleotide molecules of the present invention can further comprise, or alternatively may consist of, nucleotide sequences selected from those that naturally flank the *GRA1* ORF or gene *in situ* in *N. caninum,* and include the nucleotide sequences shown in SEQ ID NO:1 from about nt 1 to about nt 204 and from about nt 778 to about nt 1265, or as shown in SEQ ID NO:3 from about nt 1 to about nt 604, and from about nt 1305 to about nt 1774, or substantial portions thereof.

### 4.1.2. GRA2-Related Polynucleotide Molecules

References herein below to the nucleotide sequence shown in SEQ ID NO:4, and to substantial portions thereof, are intended to also refer to the corresponding nucleotide sequence and substantial portions thereof, respectively, as present in plasmid pRC5 (ATCC 209686), unless otherwise indicated. In addition, references herein below to the amino acid sequence shown in SEQ ID NO:5. and to substantial portions and peptide fragments thereof, are intended to also refer to the corresponding amino acid sequence, and substantial portions and peptide fragments thereof, respectively, encoded by the corresponding GRA2-encoding nucleotide sequence present in plasmid pRC5 (ATCC 209686), unless otherwise indicated.

Tne present invention further provides an isolated polynucleotide molecule comprising a nucleotide sequence encoding the GRA2 protein from *N. caninum.* In a preferred embodiment, the GRA2 protein has the amino acid sequence of SEQ ID NO:5. In a further preferred embodiment, the isolated GRA2-encoding polynucleotide molecule of the present invention comprises a nucleotide sequence selected from the group consisting of the nucleotide sequence of the ORF of SEQ ID NO:4, which is from about nt 25 to about nt 660, and the nucleotide sequence of the GRA2-encoding ORF of plasmid pRC5 (ATCC 209686). In a non-limiting embodiment, the isolated GRA2-encoding polynucleotide molecule of the present invention comprises the nucleotide sequence of SEQ ID NO:4.

The present invention further provides an isolated polynucleotide molecule having a nucleotide sequence that is homologous to the nucleotide sequence of a GRA2-encoding polynucleotide molecule of the present invention. The term "homologous" when used to refer to a *GRA2*-related polynucleotide molecule means a polynucleotide molecule having a nucleotide sequence: (a) that encodes the same protein as one of the aforementioned GRA2-encoding polynucleotide molecules of the present invention, but that includes one or more silent changes to the nucleotide sequence according to the degeneracy of the genetic code; or (b) that hybridizes to the complement of a polynucleotide molecule having a nucleotide sequence that encodes the amino acid sequence of the *N. caninum* GRA2 protein, under moderately stringent conditions. *i.e*., hybridization to filter-bound DNA in 0.5 M NaHPO₄, 7% SDS, 1 mM EDTA at 65°C, and washing in 0.2xSSC/0.1% SDS at 42°C (Ausubel *et al.,* 1989, above), and that is useful in practicing the present invention, as usefulness is defined above for polynucleotide molecules. In a preferred embodiment, the homologous polynucleotide molecule hybridizes to the complement of a polynucleotide molecule having a nucleotide sequence that encodes the amino acid sequence of the *N. caninum* GRA2 protein under highly stringent conditions, *i.e*., hybridization to filter-bound DNA in 0.5 M NaHPO₄, 7% SDS, 1 mM EDTA at 65°C, and washing in 0.1xSSC/0.1% SDS at 68°C (Ausubel *et al*., 1989, above), and is useful in practicing the present invention. In a more preferred embodiment, the homologous polynucleotide molecule hybridizes under highly stringent conditions to the complement of a potynucleotide molecule consisting of the nucleotide sequence of the ORF of SEQ ID NO 4, which is from about nt 25 to about nt 660, and is useful in practicing the present invention.

Polynucleotide molecules of the present invention having nucleotide sequences that are homologous to the nucleotide sequence of a GRA2-encoding polynucleotide molecule of the present invention do not include polynucleotide molecules having the native nucleotide sequence of *T. gondii* encoding a *T. gondii* GRA protein, and further have no more than about 90%, and preferably no more than about 80%, sequence identity to such a *T. gondii* polynucleotide molecule, wherein sequence identity is determined by use of the BLASTN algorithm (GenBank, NCBI).

The present invention further provides an isolated polynucleotide molecule comprising a nucleotide sequence that encodes a polypeptide that is homologous to the *N. caninum* GRA2 protein. As used herein to refer to polypeptides that are homologous to the *N. caninum* GRA2 protein, the term "homologous" refers to a polypeptide otherwise having the amino acid sequence of the *N. caninum* GRA2 protein, but in which one or more amino acid residues have been conservatively substituted with a different amino acid residue, as defined above, where the resulting polypeptide is useful in practicing the present invention, as usefulness is defined above for polypeptides.

The present invention further provides a polynucleotide molecule consisting of a substantial portion of any of the aforementioned *Neospora GRA2*-related polynucleotide molecules of the present invention. As used herein, a "substantial portion" of a GRA2-related polynucleotide molecule means a polynucleotide molecule consisting of less than the complete nucleotide sequence of the *GRA2*-related polynucleotide molecule, but comprising at least about 5%, and more preferably at least about 10%, of the nucleotide sequence of the *GRA2*-related polynucleotide molecule, and that is useful in practicing the present invention, as usefulness is defined above for polynucleotide molecules.

In addition to the nucleotide sequences of any of the aforementioned GRA2-related polynucleotide molecules, polynucleotide molecules of the present invention can further comprise, or alternatively may consist of, nucleotide sequences that naturally flank the GRA2 gene or ORF *in situ* in *N. caninum,* and include the flanking nucleotide sequences shown in SEQ ID NO:4 from about nt 1 to about nt 24, and from about nt 661 to about nt 1031, or substantial portions thereof.

### 4.1.3. SAG1-Related Polynucleotide Molecules

References herein below to the nucleotide sequence shown in SEQ ID NO:6. and to substantial portions thereof, are intended to also refer to the corresponding nucleotide sequence and substantial portions thereof, respectively, as present in plasmid pRC102 (ATCC 209687), unless otherwise indicated. In addition, references herein below to the amino acid sequence shown in SEQ ID NO:7, and to substantial portions and peptide fragments thereof, are intended to also refer to the corresponding amino acid sequence, and substantial portions and peptide fragments thereof, respectively, encoded by the corresponding SAG1-encoding nucleotide sequence present in plasmid pRC102 (ATCC 209687), unless otherwise indicated.

The present invention further provides an isolated polynucleotide molecule comprising a nucleotide sequence encoding the SAG1 protein from *N. caninum.* In a preferred embodiment, the SAG1 protein has the amino acid sequence of SEQ ID NO:7. In a further preferred embodiment, the isolated SAG1-encoding polynucleotide molecule of the present invention comprises a nucleotide sequence selected from the group consisting of the nucleotide sequence of the ORF of SEQ ID NO:6, which is from about nt 130 to about nt 1089, and the nucleotide sequence of the SAGI-encoding ORF of plasmid pRC102 (ATCC 209687). In a non-limiting embodiment, the isolated SAG1-encoding polynucleotide molecule of the present invention comprises the nucleotide sequence of SEQ ID NO:6.

The present invention further provides an isolated polynucleotide molecule having a nucleotide sequence that is homologous to the nucleotide sequence of a SAG1-encoding polynucleotide molecule of the present invention. The term "homologous" when used to refer to a SAG1-related polynucleotide molecule means a polynucleotide molecule having a nucleotide sequence: (a) that encodes the same protein as one of the aforementioned SAG1-encoding polynucleotide molecules of the present invention, but that includes one or more silent changes to the nucleotide sequence according to the degeneracy of the genetic code; or (b) that hybridizes to the complement of a polynucleotide molecule having a nucleotide sequence that encodes the amino acid sequence of the *N. caninum* SAG1 protein, under moderately stringent conditions, *i.e.,* hybridization to filter-bound DNA in 0.5 M NaHPO₄, 7% SDS, 1 mM EDTA at 65°C, and washing in 0.2xSSC/0.1% SDS at 42°C (Ausubel *et al*., 1989, above), and that is useful in practicing the present invention, as usefulness is defined above for polynucleotide molecules. In a preferred embodiment, the homologous polynucleotide molecule hybridizes to the complement of a polynucleotide molecule having a nucleotide sequence that encodes the amino acid sequence of the *N. caninum* SAG1 protein under highly stringent conditions, *i.e*., hybridization to filter-bound DNA in 0.5 M NaHPO₄, 7% SDS, 1 mM EDTA at 65°C, and washing in 0.1xSSC/0.1% SDS at 68°C (Ausubel *et al*., 1989, above), and is useful in practicing the present invention. In a more preferred embodiment, the homologous polynucleotide molecule hybridizes under highly stringent conditions to the complement of a polynucleotide molecule consisting of the nucleotide sequence of the ORF of SEQ ID NO:6, which is from about nt 130 to about nt 1089, and is useful in practicing the present invention.

Polynucleotide molecules of the present invention having nucleotide sequences that are homologous to the nucleotide sequence of a SAG1-encoding polynucleotide molecule of the present invention do not include polynucleotide molecules having the native nucleotide sequence of *T. gondii* encoding a *T. gondii* SAG1 protein, and further have no more than about 90%, and preferably no more than about 80%, sequence identity to such a *T. gondii* polynucleotide molecule, wherein sequence identity is determined by use of the BLASTN algorithm (GenBank. NCBI).

The present invention further provides an isolated polynucleotide molecule comprising a nucleotide sequence that encodes a polypeptide that is homologous to the *N. caninum* SAG1 protein. As used herein to refer to polypeptides that are homologous to the *N. caninum* SAG1 protein, the term "homologous" refers to a polypeptide otherwise having the amino acid sequence of the *N. caninum* SAG1 protein, but in which one or more amino acid residues have been conservatively substituted with a different amino acid residue, as defined above, where the resulting polypeptide is useful in practicing the present invention, as usefulness is defined above for polypeptides.

The present invention further provides a polynucleotide molecule consisting of a substantial portion of any of the aforementioned *Neospora SAG1*-related polynucleotide molecules of the present invention. As used herein, a "substantial portion" of a *SAG1*-related polynucleotide molecule means a polynucleotide molecule consisting of less than the complete nucleotide sequence of the *SAG1*-related polynucleotide molecule, but comprising at least about 5%, and more preferably at least about 10%, of the nucleotide sequence of the *SAG1*-related polynucleotide molecule, and that is useful in practicing the present invention, as usefulness is defined above for polynucleotide molecules.

In addition to the nucleotide sequences of any of the aforementioned *SAG1*-related polynucleotide molecules, polynucleotide molecules of the present invention can further comprise, or altematively may consist of, nucleotide sequences that naturally flank the *SAG1* gene or ORF *in situ* in *N. caninum,* and include the flanking nucleotide sequences shown in SEQ ID NO:6 from about nt 1 to about nt 129 and from about nt 1090 to about nt 1263, or substantial portions thereof.

### 4.1.4. MIC1-Related Polynudeotide Molecules

References herein below to the nucleotide sequences shown in SEQ ID NOS:8 and 10. and to substantial portions thereof, are intended to also refer to the corresponding nucleotide sequences and substantial portions thereof, respectively, as present in plasmid pRC340 (ATCC 209688), unless otherwise indicated. In addition, references herein below to the amino acid sequences shown in SEQ ID NO:9. and to substantial portions and peptide fragments thereof, are intended to also refer to the corresponding amino acid sequences, and substantial portions and peptide fragments thereof, respectively, encoded by the corresponding MIC1-encoding nucleotide sequence present in plasmid pRC340 (ATCC 209688), unless otherwise indicated.

The present invention further provides an isolated polynucleotide molecule comprising a nucleotide sequence encoding the MIC1 protein from *N. caninum.* In a preferred embodiment, the MIC1 protein has the amino acid sequence of SEQ ID NO:9. In a further preferred embodiment, the isolated MIC1-encoding polynucleotide molecule of the present invention comprises a nucleotide sequence selected from the group consisting of the nucleotide sequence of the ORF of SEQ ID NO:8, which is from about nt 138 to about nt 1520, the nucleotide sequence of the ORF of the *MIC1* gene, which is presented as SEQ ID NO:10, and the nucleotide sequence of the MIC1-encoding ORF of plasmid pRC340 (ATCC 209688). In a non-limiting embodiment, the isolated MIC1-encoding polynucleotide molecule of the present invention comprises a nucleotide sequence selected from the group consisting of the nucleotide sequence of SEQ ID NO:8 and SEQ ID NO:10.

The present invention further provides an isolated polynucleotide molecule having a nucleotide sequence that is homologous to the nucleotide sequence of a MIC1-encoding polynucleotide molecule of the present invention. The term "homologous" when used to refer to a MIC1-related polynucleotide molecule means a polynucleotide molecule having a nucleotide sequence: (a) that encodes the same protein as one of the aforementioned MIC1-encoding polynucleotide molecules of the present invention, but that includes one or more silent changes to the nucleotide sequence according to the degeneracy of the genetic code; or (b) that hybridizes to the complement of a polynucleotide molecule having a nucleotide sequence that encodes the amino acid sequence of the *N. caninum* MIC1 protein, under moderately stringent conditions, *i.e.,* hybridization to filter-bound DNA in 0.5 M NaHPO₄, 7% SDS, 1 mM EDTA at 65°C, and washing in 0.2xSSC/0.1% SDS at 42°C (Ausubel *et* al., 1989, above), and that is useful in practicing the present invention, as usefulness is defined above for polynucleotide molecules. In a preferred embodiment, the homologous polynucleotide molecule hybridizes to the complement of a polynucleotide molecule having a nucleotide sequence that encodes the amino acid sequence of the *N. caninum* MIC1 protein under highly stringent conditions, *i.e*., hybridization to filter-bound DNA in 0.5 M NaHPO₄, 7% SDS, 1 mM EDTA at 65°C, and washing in 0.1xSSC/0.1% SDS at 68°C (Ausubel *et al*., 1989. above). and is useful in practicing the present invention. In a more preferred embodiment, the homologous polynucleotide molecule hybridizes under highly stringent conditions to the complement of a polynucleotide molecule consisting of a nucleotide sequence selected from the group consisting of the ORF of SEQ ID NO:8 from about nt 138 to about nt 1520, and the ORF of the *MIC1* gene, which is presented as SEQ ID NO:10, and is useful in practicing the present invention.

Polynucleotide molecules of the present invention having nucleotide sequences that are homologous to the nucleotide sequence of a MIC1-encoding polynucleotide molecule of the present invention do not include polynucleotide molecules having the native nucleotide sequence of *T. gondii* encoding a *T. gondii* MIC1 protein, and further have no more than about 90%, and preferably no more than about 80%, sequence identity to such a *T. gondii* polynucleotide molecule, wherein sequence identity is determined by use of the BLASTN algorithm (GenBank, NCBI).

The present invention further provides an isolated polynucleotide molecule comprising a nucleotide sequence that encodes a polypeptide that is homologous to the *N. caninum* MIC1 protein. As used herein to refer to polypeptides that are homologous to the *N. caninum* MIC1 protein, the term "homologous" refers to a polypeptide otherwise having the amino acid sequence of the *N. caninum* MIC1 protein, but in which one or more amino acid residues have been conservatively substituted with a different amino acid residue, as defined above, where the resulting polypeptide is useful in practicing the present invention, as usefulness is defined above for polypeptides.

The present invention further provides a polynucleotide molecule consisting of a substantial portion of any of the aforementioned *Neospora MIC1*-related polynucleotide molecules of the present invention. As used herein, a "substantial portion" of a *MIC1*-related polynucleotide molecule means a polynucleotide molecule consisting of less than the complete nucleotide sequence of the *MIC1*-related polynucleotide molecule, but comprising at least about 5%, and more preferably at least about 10%, of the nucleotide sequence of the *MIC1*-related polynucleotide molecule. and that is useful in practicing the present invention, as usefulness is defined above for polynucleotide molecules.

In addition to the nucleotide sequences of any of the aforementioned *MIC1*-related polynucleotide molecules, polynucleotide molecules of the present invention can further comprise, or alternatively may consist of, nucleotide sequences that naturally flank the *MIC1* ORF or gene *in situ* in *N. caninum*, and include the nucleotide sequences as shown in SEQ ID NO:8 from about nt 1 to about 137, and from about nt 1521 to about nt 2069, or substantial portions thereof.

### 4.1.5. MAG1-Related Polynucleotide Molecules

References herein below to the nucleotide sequence shown in SEQ ID NO:11, and to substantial portions thereof, are intended to also refer to the corresponding nucleotide sequences and substantial portions thereof, respectively, as present in plasmid bd304 (ATCC 203413), unless otherwise indicated. In addition, references herein below to the amino acid sequence shown in SEQ ID NO:13, and to substantial portions and peptide fragments thereof, are intended to also refer to the corresponding amino acid sequence, and substantial portions and peptide fragments thereof, respectively, encoded by the corresponding MAG1-encoding nucleotide sequence present in plasmid bd304 (ATCC 203413), unless otherwise indicated.

The present invention further provides an isolated polynucleotide molecule comprising a nucleotide sequence encoding the MAG1 protein from *N. caninum.* In a preferred embodiment, the MAG1 protein has the amino acid sequence of SEQ ID NO:13. In a further preferred embodiment, the isolated MAG1-encoding polynucleotide molecule of the present invention comprises a nucleotide sequence selected from the group consisting of the nucleotide sequence presented in SEQ ID NO:11 from about nt 1305 to about nt 2786, a cDNA molecule prepared therefrom, such as a cDNA molecule having the ORF of SEQ ID NO:12 from about nt 122 to about nt 1381, and the nucleotide sequence of the MAGI-encoding ORF present in plasmid bd304 (ATCC 203413). The present invention further provides a polynucleotide molecule having a nucleotide sequence of any ORF present in SEQ ID NO:11. In a non-limiting embodiment, the isolated MAGI-encoding polynucleotide molecule of the present invention comprises a nucleotide sequence selected from the group consisting of the nucleotide sequence of SEQ ID NO:11 and a cDNA deduced therefrom based on the putative exon/intron boundaries.

The present invention further provides an isolated polynucleotide molecule having a nucleotide sequence that is homologous to the nucleotide sequence of a MAGI-encoding polynucleotide molecule of the present invention. The term "homologous" when used to refer to a MAG1-related polynucleotide molecule means a polynucleotide molecule having a nucleotide sequence: (a) that encodes the same protein as one of the aforementioned MAGI-encoding polynucleotide molecules of the present invention, but that includes one or more silent changes to the nucleotide sequence according to the degeneracy of the genetic code; or (b) that hybridizes to the complement of a polynucleotide molecule having a nucleotide sequence that encodes the amino acid sequence of the *N. caninum* MAG1 protein, under moderately stringent conditions, *i.e*., hybridization to filter-bound DNA in 0.5 M NaHPO₄, 7% SDS, 1 mM EDTA at 65°C, and washing in 0.2xSSC/0.1% SDS at 42°C (Ausubel *et al*., 1989. above), and that is useful in practicing the present invention, as usefulness is defined above for polynucleotide molecules. In a preferred embodiment, the homologous polynucleotide molecule hybridizes to the complement of a polynucleotide molecule having a nucleotide sequence that encodes the amino acid sequence of the *N. caninum* MAG1 protein under highly stringent conditions, *i.e*., hybridization to filter-bound DNA in 0.5 M NaHPO₄, 7% SDS, 1 mM EDTA at 65°C, and washing in 0.1xSSC/0.1% SDS at 68°C (Ausubel *et al*., 1989, above), and is useful in practicing the present invention. In a more preferred embodiment, the homologous polynucleotide molecule hybridizes under highly stringent conditions to the complement of a polynucleotide molecule consisting of a nucleotide sequence selected from the group consisting of the nucleotide sequence of the ORF of the *MAG1* gene, which is presented in SEQ ID NO:11 from about nt 1305 to about nt 2786 and a cDNA molecule prepared therefrom based on the putative exon/intron boundaries, such as a cDNA molecule having the ORF of SEQ ID NO:12 from about nt 122 to about nt 1381, and is useful in practicing the present invention.

Polynucleotide molecules of the present invention having nucleotide sequences that are homologous to the nucleotide sequence of a MAGI-encoding polynucleotide molecule of the present invention do not include polynucleotide molecules having the native nucleotide sequence of *T. gondii* encoding a *T. gondii* MAG1 protein, and further have no more than about 90%, and preferably no more than about 80%, sequence identity to such a *T. gondii* polynucleotide molecule, wherein sequence identity is determined by use of the BLASTN algorithm (GenBank, NCBI).

The present invention further provides an isolated polynucleotide molecule comprising a nucleotide sequence that encodes a polypeptide that is homologous to the *N. caninum* MAG1 protein. As used herein to refer to polypeptides that are homologous to the *N. caninum* MAG1 protein, the term "homologous" refers to a polypeptide otherwise having the amino acid sequence of the *N. caninum* MAG1 protein, but in which one or more amino acid residues have been conservatively substituted with a different amino acid residue, as defined above, where the resulting polypeptide is useful in practicing the present invention, as usefulness is defined above for polypeptides.

The present invention further provides a polynucleotide molecule consisting of a substantial portion of any of the aforementioned *Neospora MAG1*-related polynucleotide molecules of the present invention. As used herein, a "substantial portion" of a *MAG1*-related polynucleotide molecule means a polynucleotide molecule consisting of less than the complete nucleotide sequence of the *MAG1*-related polynucleotide molecule, but comprising at least about 5%, and more preferably at least about 10%, of the nucleotide sequence of the *MAG1* related polynucleotide molecule, and that is useful in practicing the present invention, as usefulness is defined above for polynucleotide molecules. For example, a substantial portion of the polynucleotide molecule of SEQ ID NO:11 can comprise putative exon 1 from about nt 704 to about nt 820, or putative exon 2 from about nt 1301 to about nt 1399, or putative exon 3 from about nt 1510 to about nt 1808, or putative exon 4 from about nt 1921 to about nt 3297.

In addition to the nucleotide sequences of any of the aforementioned *MAG1*-related polynucleotide molecules, polynucleotide molecules of the present invention can further comprise, or alternatively may consist of, nucleotide sequences that naturally flank the *MAG1* gene or ORF *in situ* in *N. caninum,* and include the nucleotide sequences as shown in SEQ ID NO:11 from about nt 1 to about nt 1304, and from about nt 2787 to about nt 4242, or that naturally flank the ORF of a cDNA molecule prepared therefrom based on the putative exon/intron boundaries, and include flanking sequences of the ORF of a cDNA molecule having the ORF of SEQ ID NO:12, from about nt 1 to about nt 121, and from about nt 1382 to about nt 1892, or substantial portions thereof.

### 4.2. Gra1/Mag1 Promoter Region

The present invention further provides a polynucleotide molecule comprising the nucleotide sequence of the *N. caninum GRA1* and *MAG1* gene promoters. During the conduct of the experimental work disclosed herein, it was determined that the *N. caninum GRA1* and *MAG1* genes disclosed herein are naturally arranged *in situ* in a head-to-head orientation with an intervening nucleotide sequence of about 577 nt in length. This intervening nucleotide sequence, which is presented in SEQ ID NO:11 from nt 127 to nt 703, represents a putative bidirectional promoter region comprising the promoters of both the *N. caninum GRA1* and *MAG1* genes.

The *GRA1*/*MAG1* bidirectional promoter region of the present invention is useful for a variety of purposes including for controlling the recombinant expression of either the *GRA1* or *MAG1* genes. or both genes, or of one or more other genes or coding sequences, in host cells *of N. caninum* or in host cells of any other species of *Neospora* or other member of the Apicomplexa. or in any other appropriate host cell. Such other genes or coding sequences can either be native or heterologous to the recombinant host cell. The promoter sequence can be fused to the particular gene or coding sequence using standard recombinant techniques as known in the art so that the promoter sequence is in operative association therewith, as "operative association" is defined below. By using the promoter, recombinant expression systems can be constructed and used to screen for compounds and transcriptional factors that can modulate the expression of the *GRA1* and *MAG1* genes of Neospora or other members of the Apicomplexa. In addition, such promoter constructs can be used to express heterologous polypeptides in *Neospora* or other members of the Apicomplexa.

### 4.3. Oligonucleotide Molecules

The present invention further provides oligonucleotide molecules that hybridize to any one of the aforementioned polynucleotide molecules of the present invention. or that hybridize to a polynucleotide molecule having a nucleotide sequence that is the complement of any one of the aforementioned polynucleotide molecules of the present invention. Such oligonucleotide molecules are preferably at least about 10 nucleotides in length, and more preferably from about 15 to about 30 nucleotides in length, and hybridize to one or more of the aforementioned polynucleotide molecules under highly stringent conditions, *i.e*., washing in 6xSSC/0.5% sodium pyrophosphate at about 37°C for ~14-base oligos, at about 48°C for ~17-base oligos, at about 55°C for ~20-base oligos, and at about 60°C for -23-base oligos. Other hybridization conditions for longer oligonucleotide molecules of the present invention can be determined by the skilled artisan using standard techniques. In a preferred embodiment, an oligonucleotide molecule of the present invention is complementary to a portion of at least one of the aforementioned polynucleotide molecules of the present invention.

Specific though non-limiting embodiments of oligonucleotide molecules useful in practicing the present invention include oligonucleotide molecules selected from the group consisting of SEQ ID NOS:14-26 and 28-34, and the complements thereof.

The oligonucleotide molecules of the present invention are useful for a variety of purposes, including as primers in amplification of a *Neospora*-specific polynucleotide molecule for use, *e.g*., in differential disease diagnosis, or to encode or act as antisense molecules useful in gene regulation. Regarding diagnostics, suitably designed primers can be used to detect the presence of *Neospora*-specific polynucleotide molecules in a sample of animal tissue or fluid, such as brain tissue, lung tissue, placental tissue, blood, cerebrospinal fluid, mucous, urine, amniotic fluid, *etc*. The production of a specific amplification product can support a diagnosis of *Neospora* infection, while lack of an amplified product can point to a lack of infection. Methods for conducting amplifications, such as the polymerase chain reaction (PCR), are described, among other places, in Innis *et al*. (eds), 1995, above; and Erlich (ed), 1992, above. Other amplification techniques known in the art, *e.g*., the ligase chain reaction, can alternatively be used. The sequences of the polynucleotide molecules disclosed herein can also be used to design primers for use in isolating homologous genes from other species or strains of *Neospora* or other members of the Apicomplexa.

### 4.4. Recombinant Expression Systems

### 4.4.1. Cloning And Expression Vectors

The present invention further provides compositions for cloning and expressing any of the polynucleotide molecules of the present invention, including cloning vectors. expression vectors, transformed host cells comprising any of said vectors, and novel strains or cell lines derived therefrom. In a preferred embodiment, the present invention provides a recombinant vector comprising a polynucleotide molecule having a nucleotide sequence encoding the GRA1. GRA2, SAG1, MIC1 or MAG1 protein of *N. caninum*. In specific though non-limiting embodiments, the present invention provides plasmid pRC77 (ATCC 209685), which encodes the *N. caninum* GRA1 protein; plasmid pRC5 (ATCC 209686), which encodes the *N. caninum* GRA2 protein; plasmid pRC102 (ATCC 209687). which encodes the *N. caninum* SAG1 protein; plasmid pRC340 (ATCC 209688), which encodes the *N. caninum* MIC1 protein; and plasmid bd304 (ATCC 203413), which encodes the *N. caninum* MAG1 protein, and which also comprises the bidirectional promoter region described above.

Recombinant vectors of the present invention. particularly expression vectors, are preferably constructed so that the coding sequence for the polynucleotide molecule of the invention is in operative association with one or more regulatory elements necessary for transcription and translation of the coding sequence to produce a polypeptide. As used herein, the term "regulatory element" includes but is not limited to nucleotide sequences that encode inducible and non-inducible promoters, enhancers, operators and other elements known in the art that serve to drive and/or regulate expression of polynucleotide coding sequences. Also, as used herein, the coding sequence is in "operative association" with one or more regulatory elements where the regulatory elements effectively regulate and allow for the transcription of the coding sequence or the translation of its mRNA, or both.

Methods are well-known in the art for constructing recombinant vectors containing particular coding sequences in operative association with appropriate regulatory elements, and these can be used to practice the present invention. These methods include *in vitro* recombinant techniques, synthetic techniques. and *in vivo* genetic recombination. See, *e.g*., the techniques described in Maniatis *et al*., 1989, above; Ausubel *et al*., 1989, above; Sambrook *et al*., 1989, above; Innis et al., 1995. above; and Erlich, 1992, above.

A variety of expression vectors are known in the art which can be utilized to express the *GRA1*, *GRA2*, *SAG1*, *MIC1*, and *MAG1* coding sequences of the present invention, including recombinant bacteriophage DNA, plasmid DNA. and cosmid DNA expression vectors containing the particular coding sequences. Typical prokaryotic expression vector plasmids that can be engineered to contain a polynucleotide molecule of the present invention include pUC8, pUC9, pBR322 and pBR329 (Biorad Laboratories, Richmond. CA), pPL and pKK223 (Pharmacia, Piscataway. NJ), pQE50 (Qiagen, Chatsworth, CA). and pGEM-T EASY (Promega, Madison, WI), among many others. Typical eukaryotic expression vectors that can be engineered to contain a polynucleotide molecule of the present invention include an ecdysone-inducible mammalian expression system (Invitrogen, Carlsbad, CA), cytomegalovirus promoter-enhancer-based systems (Promega, Madison, WI; Stratagene, La Jolla, CA; Invitrogen), and baculovirus-based expression systems (Promega), among others.

The regulatory elements of these and other vectors can vary in their strength and specificities. Depending on the host/vector system utilized, any of a number of suitable transcription and translation elements can be used. For instance, when cloning in mammalian cell systems, promoters isolated from the genome of mammalian cells, *e.g*., mouse metallothionein promoter, or from viruses that grow in these cells. *e.g*., vaccinia virus 7.5K promoter or Moloney murine sarcoma virus long terminal repeat, can be used. Promoters obtained by recombinant DNA or synthetic techniques can also be used to provide for transcription of the inserted sequence. In addition, expression from certain promoters can be elevated in the presence of particular inducers, *e.g*., zinc and cadmium ions for metallothionein promoters. Non-limiting examples of transcriptional regulatory regions or promoters include for bacteria, the β-gal promoter, the T7 promoter, the TAC promoter, λ left and right promoters, trp and lac promoters, trp-lac fusion promoters, *etc*.; for yeast, glycolytic enzyme promoters, such as ADH-I and -II promoters, GPK promoter, PGI promoter, TRP promoter, *etc*.; and for mammalian cells, SV40 early and late promoters, adenovirus major late promoters, among others. The present invention further provides a polynucleotide molecule comprising the nucleotide sequence of the promoters of both the *GRA1* and *MAG1* genes of *N. caninum,* which can be used to express any of the coding sequences of the present invention in *Neospora* or other members of the Apicomplexa.

Specific initiation signals are also required for sufficient translation of inserted coding sequences. These signals typically include an ATG initiation codon and adjacent sequences. In cases where the polynucleotide molecule of the present invention including its own initiation codon and adjacent sequences are inserted into the appropriate expression vector, no additional translation control signals may be needed. However, in cases where only a portion of a coding sequence is inserted, exogenous translational control signals, including the ATG initiation codon. may be required. These exogenous translational control signals and initiation codons can be obtained from a variety of sources, both natural and synthetic. Furthermore, the initiation codon must be in phase with the reading frame of the coding regions to ensure in-frame translation of the entire insert.

Expression vectors can also be constructed that will express a fusion protein comprising a protein or polypeptide of the present invention. Such fusion proteins can be used, *e.g*., to raise antisera against a *Neospora* protein, to study the biochemical properties of the *Neospora* protein, to engineer a *Neospora* protein exhibiting different immunological or functional properties, or to aid in the identification or purification, or to improve the stability, of a recombinantly-expressed *Neospora* protein. Possible fusion protein expression vectors include but are not limited to vectors incorporating sequences that encode β-galactosidase and trpE fusions, maltose-binding protein fusions. glutathione-S-transferase fusions and polyhistidine fusions (carrier regions). Methods are well-known in the art that can be used to construct expression vectors encoding these and other fusion proteins.

The fusion protein can be useful to aid in purification of the expressed protein. In non-limiting embodiments, *e.g*., a GRA1-maltose-binding fusion protein can be purified using amylose resin; a GRA1-glutathione-S-transferase fusion protein can be purified using glutathione-agarose beads; and a GRA1-polyhistidine fusion protein can be purified using divalent nickel resin. Alternatively, antibodies against a carrier protein or peptide can be used for affinity chromatography purification of the fusion protein. For example, a nucleotide sequence coding for the target epitope of a monoclonal antibody can be engineered into the expression vector in operative association with the regulatory elements and situated so that the expressed epitope is fused to a *Neospora* protein of the present invention. In a non-limiting embodiment, a nucleotide sequence coding for the FLAG™ epitope tag (International Biotechnologies Inc.), which is a hydrophilic marker peptide, can be inserted by standard techniques into the expression vector at a point corresponding, *e.g*., to the amino or carboxyl terminus of the GRA1 protein. The expressed GRA1 protein-FLAG™ epitope fusion product can then be detected and affinity-purified using commercially available anti-FLAG™ antibodies.

The expression vector can also be engineered to contain polylinker sequences that encode specific protease cleavage sites so that the expressed *Neospora* protein can be released from the carrier region or fusion partner by treatment with a specific protease. For example, the fusion protein vector can include a nucleotide sequence encoding a thrombin or factor Xa cleavage site, among others.

A signal sequence upstream from and in reading frame with the Neospora coding sequence can be engineered into the expression vector by known methods to direct the trafficking and secretion of the expressed protein. Non-limiting examples of signal sequences include those from α-factor, immunoglobulins. outer membrane proteins, penicillinase, and T-cell receptors, among others.

To aid in the selection of host cells transformed or transfected with a recombinant vector of the present invention, the vector can be engineered to further comprise a coding sequence for a reporter gene product or other selectable marker Such a coding sequence is preferably in operative association with the regulatory elements, as described above. Reporter genes that are useful in practicing the invention are well-known in the art and include those encoding chloramphenicol acetyltransferase (CAT), green fluorescent protein, firefly luciferase, and human growth hormone, among others. Nucleotide sequences encoding selectable markers are well-known in the art, and include those that encode gene products conferring resistance to antibiotics or anti-metabolites, or that supply an auxotrophic requirement. Examples of such sequences include those that encode thymidine kinase activity, or resistance to methotrexate. ampicillin, kanamycin, chloramphenicol, zeocin. pyrimethamine. aminoglycosides. or hygromycin, among others.

### 4.4.2. Transformation Of Host Cells

The present invention further provides transformed host cells comprising a polynucleotide molecule or recombinant vector of the present invention, and cell lines derived therefrom. Host cells useful in practicing the invention can be eukaryotic or prokaryotic cells. Such transformed host cells include but are not limited to microorganisms, such as bacteria transformed with recombinant bacteriophage DNA, plasmid DNA or cosmid DNA vectors, or yeast transformed with a recombinant vector, or animal cells, such as insect cells infected with a recombinant virus vector, *e.g.,* baculovirus, or mammalian cells infected with a recombinant virus vector, *e.g*., adenovirus or vaccinia virus, among others. For example, a strain of *E. coli* can be used, such as, *e.g*., the DH5α strain available from the ATCC, Rockville, MD, USA (Accession No. 31343), or from Stratagene (La Jolla, CA). Eukaryotic host cells include yeast cells, although mammalian cells, *e.g.,* from a mouse, hamster, cow. monkey, or human cell line, among others, can also be utilized effectively. Examples of eukaryotic host cells that can be used to express a recombinant protein of the invention include Chinese hamster ovary (CHO) cells (*e.g.,* ATCC Accession No. CCL-61), NIH Swiss mouse embryo cells NIH13T3 (*e.g*., ATCC Accession No. CRL-1658), and Madin-Darby bovine kidney (MDBK) cells (ATCC Accession No. CCL-22).

The recombinant vector of the invention is preferably transformed or transfected into one or more host cells of a substantially homogeneous culture of cells. The vector is generally introduced into host cells in accordance with known techniques, such as, *e.g*., by protoplast transformation, calcium phosphate precipitation, calcium chloride treatment, microinjection, electroporation, transfection by contact with a recombined virus, liposome-mediated transfection, DEAE-dextran transfection, transduction, conjugation, or microprojectile bombardment, among others. Selection of transformants can be conducted by standard procedures, such as by selecting for cells expressing a selectable marker, *e.g*., antibiotic resistance, associated with the recombinant expression vector.

Once an expression vector is introduced into the host cell, the integration and maintenance of the polynucleotide molecule of the present invention, either in the host cell genome or episomally, can be confirmed by standard techniques, *e.g*., by Southern hybridization analysis, restriction enzyme analysis, PCR analysis including reverse transcriptase PCR (rt-PCR), or by immunological assay to detect the expected protein product. Host cells containing and/or expressing a polynucleotide molecule of the present invention can be identified by any of at least four general approaches that are well-known in the art, including: (i) DNA-DNA, DNA-RNA, or RNA-antisense RNA hybridization, (ii) detecting the presence of "marker" gene functions; (iii) assessing the level of transcription as measured by the expression of specific mRNA transcripts in the host cell; or (iv) detecting the presence of mature polypeptide product, *e.g*., by immunoassay, as known in the art.

### 4.4.3. Expression And Purification Of Recombinant Polypeptides

Once a polynucleotide molecule of the present invention has been stably introduced into an appropriate host cell, the transformed host cell is clonally propagated, and the resulting cells are grown under conditions conducive to the maximum production of the encoded polypeptide. Such conditions typically include growing transformed cells to high density. Where the expression vector comprises an inducible promoter, appropriate induction conditions such as, *e.g*., temperature shift, exhaustion of nutrients, addition of gratuitous inducers *(e.g.,* analogs of carbohydrates, such as isopropyl-β-D-thiogalactopyranoside (IPTG)), accumulation of excess metabolic by-products, or the like, are employed as needed to induce expression.

Where the polypeptide is retained inside the host cells, the cells are harvested and lysed. and the product is substantially purified or isolated from the lysate under extraction conditions known in the art to minimize protein degradation such as, *e.g*., at 4°C, or in the presence of protease inhibitors, or both. Where the polypeptide is secreted from the host cells, the exhausted nutrient medium can simply be collected and the polypeptide substantially purified or isolated therefrom.

The polypeptide can be substantially purified or isolated from cell lysates or culture medium, as necessary, using standard methods, including but not limited to one or more of the following methods: ammonium sulfate precipitation, size fractionation, ion exchange chromatography, HPLC, density centrifugation, and affinity chromatography. If the polypeptide lacks biological activity, it can be detected as based, *e.g*., on size, or reactivity with a polypeptide-specific antibody, or by the presence of a fusion tag. For use in practicing the present invention, the polypeptide can be in an unpurified state as secreted into the culture fluid or as present in a cell lysate, but is preferably substantially purified or isolated therefrom. As used herein, a polypeptide is "substantially purified" where the polypeptide constitutes at least about 20 wt% of the protein in a particular preparation. Also. as used herein, a polypeptide is "isolated" where the polypeptide constitutes at least about 80 wt% of the protein in a particular preparation.

Thus, the present invention provides a substantially purified or isolated polypeptide encoded by a polynucleotide of the present invention. In a non-limiting embodiment, the polypeptide is a *N. caninum* protein selected from the group consisting of GRA1, GRA2, SAG1, MIC1 and MAG1 proteins. In a preferred embodiment, the *N. caninum* GRA1 protein has the amino acid sequence of SEQ ID NO:2. In another preferred embodiment, the *N. caninum* GRA2 protein has the amino acid sequence of SEQ ID NO:5 In another preferred embodiment, the *N. caninum* SAG1 protein has the amino acid sequence of SEQ ID NO:7. In another preferred embodiment, the *N. caninum* MIC1 protein has the amino acid sequence of SEQ ID NO:9. In another preferred embodiment, the *N. caninum* MAG1 protein has the amino acid sequence of SEQ ID NO:13.

The present invention further provides polypeptides that are homologous to any of the aforementioned *N. caninum* proteins, as the term "homologous" is defined above for polypeptides. Polypeptides of the present invention that are homologous to any of the aforementioned GRA1, GRA2, SAG1. MIC1 or MAG1 proteins of *N. caninum* do not include polypeptides having the native amino acid sequence of a *T. gondii* GRA, SAG, MIC or MAG protein, and further have no more than about 90%, and preferably no more than about 80%, amino acid sequence identity to such a *T. gondii* polypeptide, wherein sequence identity is determined by use of the BLASTP algorithm (GenBank, NCBI).

The present invention further provides polypeptides consisting of a substantial portion of any one of the aforementioned polypeptides of the present invention. As used herein, a "substantial portion" of a polypeptide of the present invention, or "peptide fragment," means a polypeptide consisting of less than the complete amino acid sequence of the corresponding full-length polypeptide, but comprising at least about 10%, and more preferably at least about 20%, of the amino acid sequence thereof, and that is useful in practicing the present invention, as defined above for polypeptides. Particularly preferred are peptide fragments that are immunogenic, *i.e*., capable of inducing an immune response which results in production of antibodies that react specifically against the corresponding full-length *Neospora* polypeptide.

The present invention further provides fusion proteins comprising any of the aforementioned polypeptides fused to a carrier or fusion partner as known in the art.

The present invention further provides a method of preparing any of the aforementioned polypeptides, comprising culturing a host cell transformed with a recombinant expression vector, said recombinant expression vector comprising a polynucleotide molecule comprising a nucleotide sequence encoding the particular polypeptide, which polynucleotide molecule is in operative association with one or more regulatory elements, under conditions conducive to the expression of the polypeptide, and recovering the expressed polypeptide from the cell culture.

### 4.5. Use Of Polypeptides

Once a polypeptide of the present invention of sufficient purity has been obtained. it can be characterized by standard methods, including by SDS-PAGE, size exclusion chromatography, amino acid sequence analysis, immunological activity, biological activity, *etc.* The polypeptide can be further characterized using hydrophilicity analysis (see, *e.g.,* Hopp and Woods, 1981, Proc. Natl. Acad. Sci. *USA* 78:3824), or analogous software algorithms, to identify hydrophobic and hydrophilic regions. Structural analysis can be carried out to identify regions of the polypeptide that assume specific secondary structures. Biophysical methods such as X-ray crystallography (Engstrom, 1974, Biochem. Exp. Biol. 11: 7-13), computer modeling (Fletterick and Zoller (eds), 1986, in: Current Communications in Molecular Biology, Cold Spring Harbor Laboratory, Cold Spring Harbor, NY), and nuclear magnetic resonance (NMR) can be used to map and study potential sites of interaction between the polypeptide and other putative interacting proteins/receptors/molecules. Information obtained from these studies can be used to design deletion mutants and vaccine compositions, and to design or select therapeutic or pharmacologic compounds that can specifically block the biological function of the polypeptide *in vivo*.

Polypeptides of the present invention are useful for a variety of purposes, including as components of vaccine compositions to protect mammals against neosporosis; or as diagnostic reagents, *e.g*., using standard techniques such as ELISA assays, to screen for *Neospora*-specific antibodies in blood or serum samples from animals; or as antigens to raise polyclonal or monoclonal antibodies, as described below, which antibodies are useful as diagnostic reagents, *e.g*., using standard techniques such as Western blot assays, to screen for *Neospora*-specific proteins in cell, tissue or fluid samples from an animal.

### 4.6. Analogs And Derivatives Of Polypeptides

Any polypeptide of the present invention can be modified at the protein level to improve or otherwise alter its biological or immunological characteristics. One or more chemical modifications of the polypeptide can be carried out using known techniques to prepare analogs therefrom, including but not limited to any of the following: substitution of one or more L-amino acids of the polypeptide with corresponding D-amino acids, amino acid analogs, or amino acid mimics, so as to produce, *e.g*., carbazates or tertiary centers; or specific chemical modification, such as. *e.g*., proteolytic cleavage with trypsin, chymotrypsin, papain or V8 protease, or treatment with NaBH₄ or cyanogen bromide, or acetylation, formylation, oxidation or reduction, *etc.* Alternatively or additionally, polypeptides of the present invention can be modified by genetic recombination techniques.

A polypeptide of the present invention can be derivatized by conjugation thereto of one or more chemical groups, including but not limited to acetyl groups, sulfur bridging groups, glycosyl groups, lipids, and phosphates, and/or by conjugation to a second polypeptide of the present invention, or to another protein, such as, *e.g*., serum albumin, keyhole limpet hemocyanin, or commercially activated BSA, or to a polyamino acid (*e.g*., polylysine), or to a polysaccharide, (*e.g*., sepharose, agarose, or modified or unmodified celluloses), among others. Such conjugation is preferably by covalent linkage at amino acid side chains and/or at the N-terminus or C-terminus of the polypeptide. Methods for carrying out such conjugation reactions are well-known in the field of protein chemistry.

Derivatives useful in practicing the claimed invention also include those in which a water-soluble polymer such as, *e.g*., polyethylene glycol, is conjugated to a polypeptide of the present invention, or to an analog or derivative thereof, thereby providing additional desirable properties while retaining, at least in part, the immunogenicity of the polypeptide. These additional desirable properties include, *e.g.,* increased solubility in aqueous solutions, increased stability in storage, increased resistance to proteolytic degradation, and increased *in vivo* half-life. Water-soluble polymers suitable for conjugation to a polypeptide of the present invention include but are not limited to polyethylene glycol homopolymers, polypropylene glycol homopolymers, copolymers of ethylene glycol with propylene glycol, wherein said homopolymers and copolymers are unsubstituted or substituted at one end with an alkyl group, polyoxyethylated polyols, polyvinyl alcohol, polysaccharides, polyvinyl ethyl ethers, and α,β-poly[2-hydroxyethyl]-DL-aspartamide. Polyethylene glycol is particularly preferred. Methods for making water-soluble polymer conjugates of polypeptides are known in the art and are described in, among other places, U.S. Patent 3,788,948; U.S. Patent 3,960,830; U.S. Patent 4,002,531; U.S. Patent 4,055,635; U.S. Patent 4,179,337; U.S. Patent 4,261,973; U.S. Patent 4,412,989; U.S. Patent 4,414,147; U.S. Patent 4,415,665; U.S. Patent 4,609,546; U.S. Patent 4,732,863; U.S. Patent 4,745,180; European Patent (EP) 152,847; EP 98,110; and Japanese Patent 5,792,435, which patents are incorporated herein by reference.

### 4.7. Antibodies

The present invention further provides isolated antibodies directed against a polypeptide of the present invention. In a preferred embodiment, antibodies can be raised against a GRA1. GRA2, SAG1, MIC1 or MAG1 protein from *N*. *caninum* using known methods. Various host animals selected from pigs. cows, horses, rabbits, goats, sheep, or mice, can be immunized with a partially or substantially purified, or isolated, *N. caninum* protein, or with a homolog, fusion protein, substantial portion, analog or derivative thereof, as these are described above. An adjuvant, such as described below, can be used to enhance antibody production.

Polyclonal antibodies can be obtained and isolated from the serum of an immunized animal and tested for specificity against the antigen using standard techniques. Alternatively, monoclonal antibodies can be prepared and isolated using any technique that provides for the production of antibody molecules by continuous cell lines in culture. These include but are not limited to the hybridoma technique originally described by Kohler and Milstein (Nature, 1975, 256: 495-497); the human B-cell hybridoma technique (Kosbor *et al*., 1983, Immunology Today 4:72; Cote *et al*., 1983, Proc. Natl. Acad. Sci. *USA* 80: 2026-2030); and the EBV-hybridoma technique (Cole *et al*., 1985, Monoclonal Antibodies and Cancer Therapy. Alan R. Liss, Inc., pp. 77-96). Alternatively, techniques described for the production of single chain antibodies (see, *e.g*., U.S. Patent 4,946,778) can be adapted to produce *N. caninum* antigen-specific single chain antibodies. These publications are incorporated herein by reference.

Antibody fragments that contain specific binding sites for a polypeptide of the present invention are also encompassed within the present invention, and can be generated by known techniques. Such fragments include but are not limited to F(ab')₂ fragments, which can be generated by pepsin digestion of an intact antibody molecule, and Fab fragments, which can be generated by reducing the disulfide bridges of the F(ab')₂ fragments. Alternatively, Fab expression libraries can be constructed (Huse *et al*., 1989, Science 246: 1275-1281) to allow rapid identification of Fab fragments having the desired specificity to the *N. caninum* protein.

Techniques for the production and isolation of monoclonal antibodies and antibody fragments are well-known in the art, and are additionally described, among other places, in Harlow and Lane, 1988, Antibodies: A Laboratory Manual, Cold Spring Harbor Laboratory, and in J. W. Goding, 1986, Monoclonal Antibodies: Principles and Practice, Academic Press, London, which are incorporated herein by reference.

### 4.8. Targeted Mutation Of Neospora Genes

Based on the disclosure of the polynucleotide molecules of the present invention, genetic constructs can be prepared for use in disabling or otherwise mutating a *Neospora GRA1*, *GRA2*, *SAG1*, *MIC1* or *MAG1* gene (which genes are hereinafter referred to collectively or individually as the "*Neospora* genes" or a "*Neospora* gene," respectively). Each of the *Neospora* genes can be mutated using an appropriately designed genetic construct in combination with genetic techniques now known or to be developed in the future. For example, a *Neospora* gene can be mutated using a genetic construct of the present invention that functions to: (a) delete all or a portion of the coding sequence or regulatory sequence of the *Neospora* gene; or (b) replace all or a portion of the coding sequence or regulatory sequence of the *Neospora* gene with a different nucleotide sequence; or (c) insert into the coding sequence or regulatory sequence of the *Neospora* gene one or more nucleotides, or an oligonucleotide molecule, or polynucleotide molecule, which can comprise a nucleotide sequence from *Neospora* or from a heterologous source; or (d) carry out some combination of (a). (b) and (c).

*Neospora* cells in which a *Neospora* gene has been mutated are useful in practicing the present invention where mutating the gene reduces the pathogenicity of the *Neospora* cells carrying the mutated gene compared to cells of the same strain of *Neospora* where the gene has not been so mutated, and where such *Neospora* cells carrying the disabled gene can be used in a vaccine composition, particularly in a modified live vaccine, to induce or contribute to the induction of, a protective response in a mammal against neosporosis. In a preferred embodiment, the mutation serves to partially or completely disable the Neospora gene, or partially or completely disable the protein encoded by the Neospora gene. In this context, a *Neospora* gene or protein is considered to be partially or completely disabled if either no protein product is made (for example, the gene is deleted), or a protein product is made that can no longer carry out its normal biological function or can no longer be transported to its normal cellular location, or a product is made that carries out its normal biological function but at a significantly reduced rate, or if such mutation results in a detectable decrease in the pathogenicity of cells of a pathogenic strain of *Neospora* wherein the gene has been so mutated compared to cells of the same strain but in which the gene has not be so mutated.

In a non-limiting embodiment, a genetic construct of the present invention is used to mutate a wild-type *Neospora* gene by replacement of the coding sequence of the wild-type gene, or a promoter or other regulatory region thereof, or a portion thereof, with a different nucleotide sequence such as, *e.g*., a mutated coding sequence or mutated regulatory region, or portion thereof. Mutated *Neospora* gene sequences for use in such a genetic construct can be produced by any of a variety of known methods, including by use of error-prone PCR, or by cassette mutagenesis. For example, oligonucleotide-directed mutagenesis can be employed to alter the coding sequence or promoter sequence of a wild-type *Neospora* gene in a defined way, *e.g*., to introduce a frame-shift or a termination codon at a specific point within the sequence. Alternatively or additionally, a mutated nucleotide sequence for use in the genetic construct of the present invention can be prepared by insertion into the coding sequence or promoter sequence of one or more nucleotides, oligonucleotide molecules or polynucleotide molecules, or by replacement of a portion of the coding sequence or promoter sequence with one or more different nucleotides, oligonucleotide molecules or polynucleotide molecules. Such oligonucleotide molecules or polynucleotide molecules can be obtained from any naturally occurring source or can be synthetic. The inserted sequence can serve simply to disrupt the reading frame of the *Neospora* gene, or can further encode a heterologous gene product such as a selectable marker.

Alternatively or additionally, random mutagenesis can be used to produce a mutated *Neospora* gene sequence for use in a genetic construct of the present invention. Random mutagenesis can be carried out by any techniques now known or to be developed in the future such as, *e.g.,* by exposing cells carrying a *Neospora* gene to ultraviolet radiation or x-rays, or to chemical mutagens such as N-methyl-N'-nitrosoguanidine, ethyl methane sulfonate, nitrous acid or nitrogen mustards, and then selecting for cells carrying a mutation in the particular gene. See, *e.g.*, Ausubel, 1989, above, for a review of mutagenesis techniques.

Mutations to produce modified *Neospora* cells that are useful in practicing the present invention, as defined above, can occur anywhere in the *Neospora* gene, including in the ORF, or in the promoter or other regulatory region, or in any other sequences that naturally comprise the gene or ORF. Such *Neospora* cells include mutants in which a modified form of the protein normally encoded by the *Neospora* gene is produced, or in which no protein normally encoded by the *Neospora* gene is produced, and can be null, conditional or leaky mutants.

Alternatively, a genetic construct of the present invention can comprise nucleotide sequences that naturally flank the *Neospora* gene or ORF *in situ,* such as those presented in SEQ ID NOS:1, 3, 4, 6, 8, 10, 11 and 12, with only a portion or no nucleotide sequences from the coding region of the gene itself. Such a genetic construct would be useful, *e.g*., to delete the entire *Neospora* gene or ORF.

In a preferred embodiment, a genetic construct of the present invention comprises a polynucleotide molecule that can be used to disable a *Neospora* gene, comprising: (a) a polynucleotide molecule having a nucleotide sequence that is otherwise the same as a nucleotide sequence encoding a GRA1, GRA2, SAG1, MIC1 or MAG1 protein from *N. caninum,* but which nucleotide sequence further comprises one or more disabling mutations; or (b) a polynucleotide molecule comprising a nucleotide sequence that naturally flanks the ORF of a *Neospora* gene *in situ.* Once transformed into cells of a strain of *Neospora,* the polynucleotide molecule of the genetic construct is specifically targeted to the particular *Neospora* gene by homologous recombination, and thereby either replaces the gene or portion thereof or inserts into the gene. As a result of this recombination event, the *Neospora* gene otherwise native to that particular strain of *Neospora* is disabled.

Methods for carrying out homologous gene replacement in parasitic protozoans are known in the art, and are described, among other places, in Cruz and Beverley, 1990. Nature 348:171-173; Cruz *et al*., 1991, Proc. Natl. Acad. Sci. USA 88:7170-7174, Donald and Roos. 1994, Mol. Biochem. Parasitol. 63:243-253; and Titus *et al*., 1995, Proc. Natl. Acad. Sci. USA 92:10267-10271, all of which are incorporated herein by reference.

For targeted gene mutation through homologous recombination, the genetic construct is preferably a plasmid, either circular or linearized, comprising a mutated nucleotide sequence as described above. In a non-limiting embodiment, at least about 200 nucleotides of the mutated sequence are used to specifically direct the genetic construct of the present invention to the particular targeted *Neospora* gene for homologous recombination, although shorter lengths of nucleotides can also be effective. In addition, the plasmid preferably compnses an additional nucleotide sequence encoding a reporter gene product or other selectable marker that is constructed so that it will insert into the *Neospora* genome in operative association with the regulatory element sequences of the native *Neospora* gene to be disrupted. Reporter genes that can be used in practicing the invention are well-known in the art and include those encoding CAT, green fluorescent protein, and β-galactosidase, among others. Nucleotide sequences encoding selectable markers are also well-known in the art, and include those that encode gene products conferring resistance to antibiotics or anti-metabolites. or that supply an auxotrophic requirement. Examples of such sequences include those that encode pyrimethamine resistance, or neomycin phosphotransferase (which confers resistance to aminoglycosides), or hygromycin phosphotransferase (which confers resistance to hygromycin).

Methods that can be used for creating the genetic constructs of the present invention are well-known in the art, and include *in vitro* recombinant techniques, synthetic techniques, and *in vivo* genetic recombination, as described, among other places, in Maniatis *et al*., 1989, above; Ausubel *et al*., 1989, above; Sambrook *et al*., 1989, above; Innis *et al*., 1995, above; and Erfich, 1992, above.

*Neospora* cells can be transformed or transfected with a genetic construct of the present invention in accordance with known techniques, such as, *e.g*., by electroporation. Selection of transformants can be carried out using standard techniques, such as by selecting for cells expressing a selectable marker associated with the construct. Identification of transtormants in which a successful recombination event has occurred and the particular target gene has been disabled can be carried out by genetic analysis, such as by Southern blot analysis, or by Northern analysis to detect a lack of mRNA transcripts encoding the particular protein, or by the appearance of a novel phenotype, such as reduced pathogenicity, or cells lacking the particular protein, as determined, *e.g*., by immunological analysis, or some combination thereof.

*Neospora* cells that can be modified according to the present invention are preferably tachyzoites, but can altematively be bradyzcites or oocysts. Although cells in certain stages of the *Neospora* life cycle are diploid, tachyzoites are haploid. Thus, the use of tachyzoites in the production of modified *Neospora* cells expressing the appropriate mutant phenotype is preferred because tachyzoites require only a singe successful recombination event to disrupt the particular *Neospora* gene. Alternatively, in diploid cells of *Neospora*, two alleles must be disrupted for each gene. This can be accomplished by sequentially targeting the first allele and then the second allele with genetic constructs bearing two different selectable markers.

In a further non-limiting embodiment, the genetic construct of the present invention can additionally comprise a different gene or coding region from *Neospora* or from a different pathogen that infects the animal, which gene or coding region encodes an antigen useful to induce, or contribute to the induction of, a separate and distinct protective immune response in the animal upon vaccination with the modified live *Neospora* cells of the present invention. This additional gene or coding region can be further engineered to contain a signal sequence that leads to secretion of the encoded antigen from the modified live *Neospora* cell, thereby allowing for the antigen to be displayed to the immune system of the vaccinated animal.

The present invention thus provides modified live *Neospora* cells in which the *GRA1*, *GRA2*, *SAG1*, *MIC1* or *MAG1* gene has been mutated. The present invention further provides modified live *Neospora* cells in which a combination of two or more of the *GRA1*, *GRA2*, *SAG1*, *MIC1*, and *MAG1* genes have been mutated, which cells can be prepared using the general methods presented above. In addition, the present invention provides a method of preparing modified live *Neospora* cells, comprising: (a) transforming cells of *Neospora* with a genetic construct of the invention; (b) selecting transformed cells in which the *GRA1*, *GRA2*, *SAG1*, *MIC1*, or *MAG1* gene has been mutated by the genetic construct; and (c) selecting from among the cells of step (b) those cells that can be used in a vaccine to protect a mammal against neosporosis.

### 4.9. Culturing Neospora Cells

*Neospora* cells for use in the present invention can be cultured and maintained *in vitro* by infecting any receptive host cell line, preferably a mammalian cell line, with tachyzoites according to known techniques described in the art. Mammalian cell lines in which tachyzoites of *Neospora* can be cultured include, *e.g*., human foreskin fibroblasts (Lindsay *et al*., 1993, Am. J. Vet. Res. 54:103-106), bovine cardiopulmonary aortic endothelial cells (Marsh *et al*., 1995, above), bovine monocytes (Lindsay and Dubey, 1989, above), and monkey kidney cells, among others. For example, tachyzoites of *N. caninum* can be cultured in monolayers of Hs68 human foreskin fibroblast cells (ATCC Accession No. CRL-1635) (Lindsay *et al*., 1993. above); and MARC145 monkey kidney cells infected with tachyzoites of *N. caninum* strain NC-1 for use in the present invention are on deposit with the ATCC (Accession No 12231). Bradyzoites can be similarly cultured and manipulated.

Mammalian cell cultures can be grown, and cell cultures that have been infected with *Neospora* cells can be maintained, in any of several types of culture media described in the art. For example, stationary monolayer cultures of bovine cardiopulmonary aortic endothelial cells infected with tachyzoites of *N. caninum* can be grown in Dulbecco's Minimum Essential Medium (DMEM; Gibco Laboratories. N.Y.), supplemented with 10% (v/v) heat-inactivated fetal bovine serum (FBS) or adult equine serum (ES), 2 mM L-glutamine, 50 U/ml penicillin, and 50 µg/ml streptomycin (Conrad *et al*., 1993, above). Monolayers of Hs68 human foreskin fibroblast cells can be maintained in RPMI 1640 containing 2% (v/v) FBS, 1.0 mM sodium pyruvate, 1 x 10⁴ U/ml penicillin, 1 x 10⁴ µg/ml streptomycin, 5 x 10⁻² mM 2-mercaptoethanol and 0.3 mg/ml L-glutamine (maintenance medium). Monolayer cultures of Hs68 human foreskin fibroblast cells infected with *Neospora* can be maintained in identical media, but in which the FBS is increased to 10% (v/v) (growth medium).

*Neospora*-infected monolayer cultures of mammalian cells are typically maintained under standard tissue culture conditions such as, *e.g.,* at 37°C and 5% CO₂. Tachyzoites are typically passaged to uninfected monolayer cultures when 70-90% of the mammalian cells in the culture have become infected, which can be determined microscopically using standard techniques. Tachyzoites can be collected from the infected mammalian cell cultures by lysing the host cells using any standard technique and collecting the tachyzoites, *e.g.*, by filtration or by centrifugation.

Modified live *Neospora* cells of the present invention can also be cultured in mammalian cells, as described above.

### 4.10. Anti-Neospora Vaccines

The present invention further provides a vaccine against neosporosis, comprising an immunologically effective amount of one or more proteins or polypeptides of the present invention, and a veterinarily acceptable carrier. In a preferred embodiment, the vaccine comprises a *N. caninum* protein selected from the group consisting of GRA1, GRA2, SAG1. MIC1 and MAG1.

The present invention further provides a vaccine against neosporosis, comprising an immunologically effective amount of one or more polynucleotide molecules of the present invention, and a veterinarily acceptable carrier. In a preferred embodiment, the vaccine comprises a polynucleotide molecule having a nucleotide sequence encoding a *N. caninum* protein selected from the group consisting of GRA1, GRA2, SAG1, MIC1, and MAG1.

The present invention further provides a vaccine against neosporosis, comprising an immunologically effective amount of modified *Neospora* cells of the present invention, and a veterinarily acceptable carrier. In a preferred embodiment, the modified *Neospora* cells for use in the vaccine of the present invention are live cells of *N. caninum* which express a *GRA1*⁻, *GRA2*⁻, *SAG1*⁻, *MIC1*⁻, or *MAG1*⁻ phenotype. Alternatively, the vaccine of the present invention can comprise any of such modified *Neospora* cells of the present invention that have been inactivated. Inactivation of modified *Neospora* cells can be carried out using any techniques Known in the art, including by chemical treatment, such as with binary ethylenimine (BEI), or beta-propiolactone, or by freeze-thawing or heat treatment, or by homogenization of cells, or by a combination of these types of techniques. Vaccines prepared from homogenized, modified *Neospora* cells can consist of either the entire unfractionated cell homogenate, or an immunologically effective subfraction thereof.

As used herein, the term "immunologically effective amount" refers to that amount of antigen, *e.g*., protein, polypeptide. polynucleotide molecule, or modified cells, capable of inducing a protective response against neosporosis when administered to a member of a mammalian species after either a single administration, or after multiple administrations.

The phrase "capable of inducing a protective response" is used broadly herein to include the induction or enhancement of any immune-based response in the animal in response to vaccination, including either an antibody or cell-mediated immune response, or both, that serves to protect the vaccinated animal against neosporosis. The terms "protective response" and "protect" as used herein refer not only to the absolute prevention of neosporosis or absolute prevention of infection by a neosporosis-causing pathogen, but also to any detectable reduction in the degree or rate of infection by such a pathogen, or any detectable reduction in the severity of the disease or any symptom or condition resulting from infection by the pathogen. including, *e.g*., any detectable reduction in the rate of formation, or in the absolute number, of lesions formed in one or more tissues, or any detectable reduction in the occurrence of abortion, or the transmission of infection from a pregnant mammal to its fetus or from a mammal parent to its offspring, in the vaccinated animal as compared to an unvaccinated infected animal of the same species.

In a further preferred embodiment, the vaccine of the present invention is a combination vaccine for protecting a mammal against neosporosis and, optionally, one or more other diseases or pathological conditions that can afflict the mammal, which combination vaccine comprises an immunologically effective amount of a first component comprising a polypeptide, polynucleotide molecule, or modified *Neospora* cells of the present invention; an immunologically effective amount of a second component that is different from the first component, and that is capable of inducing, or contributing to the induction of, a protective response against a disease or pathological condition that can afflict the mammal; and a veterinarily acceptable carrier.

The second component of the combination vaccine is selected based on its ability to induce, or contribute to the induction of, a protective response against either neosporosis or another disease or pathological condition that can afflict members of the mammalian species, as known in the art. Any antigenic component now known in the art, or to be determined in the future, to be useful in a vaccine composition in the particular mammalian species can be used as the second component of the combination vaccine. Such antigenic components include but are not limited to those that provide protection against pathogens selected from the group consisting of bovine herpes virus (syn., infectious bovine rhinotracheitis), bovine respiratory syncitial virus, bovine viral diarrhea virus, parainfluenza virus types I, II, or III, *Leptospira* spp., *Campylobacter* spp., *Staphylococcus aureus*, *Streptococcus agalactiae*, *Mycoplasma* spp., *Klebsiella* spp., *Salmonella* spp., rotavirus, coronavirus, rabies, *Pasteurella hemolytica, Pasteurella multocida*, *Clostridia* spp., *Tetanus* toxoid, *E. coli*, *Cryptosporidium* spp., *Eimeria* spp., *Trichomonas* spp., and other eukaryotic parasites, among others.

In a non-limiting embodiment, the combination vaccine of the present invention comprises a combination of two or more components selected from the group consisting of an immunologically effective amount of a protein or polypeptide of the present invention, an immunologically effective amount of a polynucleotide molecule of the present invention, and an immunologically effective amount of modified *Neospora* cells of the present invention. In a preferred embodiment, the combination vaccine of the present invention comprises a combination of two or more components selected from the group consisting of *N. caninum* GRA1, GRA2, SAG1, MIC1, and MAG1 proteins, polynucleotide molecules encoding any of the *N. caninum* GRA1, GRA2, SAG1. MIC1, and MAG1 proteins, and modified live *Neospora* cells exhibiting any of the *GRA1*⁻, *GRA2*⁻, *SAG1*⁻, *MIC1*⁻, and *MAG1*⁻ phenotypes.

The vaccines of the present invention can further comprise one or more additional immunomodulatory components including, *e.g*., an adjuvant or cytokine, as described below.

The present invention further provides a method of preparing a vaccine against neosporosis, comprising combining an immunologically effective amount of a *N*. *caninum* protein or polypeptide, or polynucleotide molecule, or modified *Neospora* cells of the present inventon, with a veterinarily acceptable carrier, in a form suitable for administration to a mammal. In a preferred embodiment, the protein is a *N. caninum* protein selected from the group consisting of GRA1, GRA2, SAG1, MIC1 and MAG1: the polynucleotide molecule preferably comprises a nucleotide sequence encoding a *N. caninum* protein selected from the group consisting of GRA1, GRA2, SAG1, MIC1 and MAG1; and the modified *Neospora* cells preferably are live cells that exhibit a phenotype selected from the group consisting of *GRA1*⁻. *GRA2*⁻, *SAG1*⁻, *MIC1*⁻, and *MAG1*⁻.

A vaccine comprising modified live *Neospora* cells of the present invention can be prepared using an aliquot of culture fluid containing said *Neospora* cells, either free in the medium or residing in mammalian host cells, or both, and can be administered directly or in concentrated form to the mammal. Alternatively, modified live *Neospora* cells can be combined with a veterinarily acceptable carrier, with or without an immunomodulatory agent, selected from those known in the art and appropriate to the chosen route of administration, preferably where at least some degree of viability of the modified live *Neospora* cells in the vaccine composition is maintained. Modified *Neospora* cells that can be used in the vaccine of the present invention are preferably tachyzoites, but can alternatively be bradyzoites or oocysts, or some combination thereof.

Vaccine compositions of the present invention can be formulated following accepted convention to include veterinarily acceptable carriers, such as standard buffers, stabilizers, diluents, preservatives, and/or solubilizers, and can also be formulated to facilitate sustained release. Diluents include water, saline, dextrose, ethanol, glycerol. and the like. Additives for isotonicity include sodium chloride, dextrose, mannitol, sorbitol, and lactose, among others. Stabilizers include albumin, among others. Suitable other vaccine vehicles and additives, including those that are particularly useful in formulating modified live vaccines, are known or will be apparent to those skilled in the art,. See, *e.g.,* Remington's Pharmaceutical Science, 18th ed., 1990, Mack Publishing, which is incorporated herein by reference.

The vaccine of the present invention can further comprise one or more additional immunomodulatory components such as, *e.g*., an adjuvant or cytokine, among others. Non-limiting examples of adjuvants that can be used in the vaccine of the present invention include the RIBI adjuvant system (Ribi Inc., Hamilton, MT), alum, mineral gels such as aluminum hydroxide gel, oil-in-water emulsions, water-in-oil emulsions such as, *e.g*., Freund's complete and incomplete adjuvants, Block co polymer (CytRx, Atlanta GA), QS-21 (Cambridge Biotech Inc., Cambridge MA), SAF-M (Chiron, Emeryville CA), AMPHIGEN® adjuvant, saponin, Quil A or other saponin fraction, monophosphoryl lipid A, and Avridine lipid-amine adjuvant. Specific non-limiting examples of oil-in-water emulsions useful in the vaccine of the invention include modified SEAM62 and SEAM 1/2 formulations. Modified SEAM62 is an oil-in-water emulsion containing 5% (v/v) squalene (Sigma), 1% (v/v) SPAN® 85 detergent (ICI Surfactants), 0.7% (v/v) TWEEN® 80 detergent (ICI Surfactants), 2.5% (v/v) ethanol, 200 µg/ml Quil A, 100 µg/ml cholesterol, and 0.5% (v/v) lecithin. Modified SEAM 1/2 is an oil-in-water emulsion comprising 5% (v/v) squalene, 1% (v/v) SPAN® 85 detergent, 0.7% (v/v) Tween 80 detergent, 2.5% (v/v) ethanol, 100 µg/ml Quil A, and 50 µg/ml cholesterol. Other immunomodulatory agents that can be included in the vaccine include, e.g., one or more interleukins, interferons. or other known cytokines. Where the vaccine comprises modified live *Neospora* cells, the adjuvant is preferably selected based on the ability of the resulting vaccine formulation to maintain at least some degree of viability of the modified live *Neospora* cells.

Where the vaccine composition comprises modified live *Neospora* cells, the vaccine can be stored cold or frozen. Where the vaccine composition instead comprises a protein, polypeptide, polynucleotide molecule, or inactivated modified *Neospora* cells of the present invention, the vaccine may be stored frozen, or in lyophilized form to be rehydrated prior to administration using an appropriate diluent.

The vaccine of the present invention can optionally be formulated for sustained release of the antigen. Examples of such sustained release formulations include antigen in combination with composites of biocompatible polymers, such as, *e.g*., poly(lactic acid), poly(lactic-co-glycolic acid), methylcellulose, hyaluronic acid, collagen and the like. The structure, selection and use of degradable polymers in drug delivery vehicles have been reviewed in several publications, including A. Domb *et al*., 1992, Polymers for Advanced Technologies 3: 279-292, which is incorporated herein by reference. Additional guidance in selecting and using polymers in pharmaceutical formulations can be found in the text by M. Chasin and R. Langer (eds), 1990, "Biodegradable Polymers as Drug Delivery Systems" in: Drugs and the Pharmaceutical Sciences, Vol. 45, M. Dekker, NY, which is also incorporated herein by reference. Alternatively, or additionally, the antigen can be microencapsulated to improve administration and efficacy. Methods for microencapsulating antigens are well-known in the art, and include techniques described, e.g., in U.S. Patent 3,137,631; U.S. Patent 3,959,457; U.S. Patent 4,205,060; U.S. Patent 4,606,940; U.S. Patent 4,744,933; U.S. Patent 5,132,117; and International Patent Publication WO 95/28227, all of which are incorporated herein by reference.

Liposomes can also be used to provide for the sustained release of antigen. Details concerning how to make and use liposomal formulations can be found in, among other places, U.S. Patent 4,016,100; U.S. Patent 4,452,747; U.S. Patent 4,921,706; U.S. Patent 4,927,637; U.S. Patent 4,944,948; U.S. Patent 5,008,050; and U.S. Patent 5,009,956, all of which are incorporated herein by reference.

The present invention further provides a method of vaccinating a mammal against neosporosis, comprising administering to the mammal an immunologically effective amount of a vaccine of the present invention. The vaccine is preferably administered parenterally, *e.g*., either by subcutaneous or intramuscular injection. However, the vaccine can alternatively be administered by intraperitoneal or intravenous injection, or by other routes, including, *e.g*., orally, intranasally, rectally, vaginally, infra-ocularly, or by a combination of routes, and also by delayed release devices as known in the art. The skilled artisan will be able to determine the most optimal route of vaccine administration, and will also recognize acceptable formulations for the vaccine composition according to the chosen route of administration.

An effective dosage can be determined by conventional means, starting with a low dose of antigen, and then increasing the dosage while monitoring the effects. Numerous factors may be taken into consideration when determining an optimal dose per animal. Primary among these is the species, size, age and general condition of the animal, the presence of other drugs in the animal, the virulence of a particular species or strain of *Neospora* against which the animal is being vaccinated, and the like. The actual dosage is preferably chosen after consideration of the results from other animal studies.

The dose amount of a Neospora protein or polypeptide of the present invention in a vaccine of the present invention preferably ranges from about 10 µg to about 10 mg, more preferably from about 50 µg to about 1 mg, and most preferably from about 100 µg to about 0.5 mg. The dose amount of a *Neospora* polynucleotide molecule of the present invention in a vaccine of the present invention preferably ranges from about 50 µg to about 1 mg. The dose amount of modified *Neospora* cells of the present invention in a vaccine of the present invention preferably ranges from about 1 x 10³ to about 1 x 10⁸ cells/ml, and more preferably from about 1 x 10⁵ to about 1 x 10⁷ cells/ml. A suitable dosage size ranges from about 0.5 ml to about 10 ml, and more preferably from about 1 ml to about 5 ml. The dose amounts of these antigens are also applicable to combination vaccines of the present invention. Where the second component of the combination vaccine is an antigen other than a *Neospora* protein, polypeptide, polynucleotide or modified cell of the present invention, the dose amount of the second component for use in the combination vaccine can be determined from prior vaccine applications of that second component, as known in the art.

The vaccine of the present invention is useful to protect mammals against neosporosis. As used herein, the term "mammal" refers to any mammalian species that can be protected against neosporosis using the vaccine of the invention, including dogs, cows, goats, sheep and horses, among others. The vaccine of the invention can be administered at any time during the life of a particular animal depending upon several factors including, *e.g.*, the timing of an outbreak of neosporosis among other animals, *etc*. The vaccine can be administered to animals of weaning age or younger, or to more mature animals, *e.g*., as a pre-breeding vaccine to protect against *Neospora*-related congenital disease or abortion. Effective protection may require only a primary vaccination, or one or more booster vaccinations may also be needed. One method of detecting whetrer adequate immune protection has been achieved is to determine seroconversion and antibody titer in the animal after vaccination. The timing of vaccination and the number of boosters, if any, will preferably be determined by a veterinarian based on analysis of all relevant factors, some of which are described above.

The present invention further provides a kit for vaccinating a mammal against neosporosis, comprising a container having an immunologically effective amount of a polypeptide, polynucleotide molecule, or modified *Neospora* cells of the present invention, or a combination thereof. The kit can optionally comprise a second container having a veterinarily acceptable carrier or diluent. In a preferred embodiment, the polypeptide is selected from the group consisting of GRA1. GRA2, SAG1, MIC1 and MAG1 proteins of *N. caninum;* the polynucleotide molecule preferably has a nucleotide sequence that encodes a *N. caninum* protein selected from the group consisting of GRA1, GRA2, SAG1, MIC1, and MAG1; and the modified *Neospora* cells preferably are live cells that express a *GRA1*⁻*, GRA2*⁻, *SAG1*⁻, *MIC1*⁻ or *MAG1*⁻ phenotype.

The following example is illustrative only, and is not intended to limit the scope of the present invention.

### 5. EXAMPLE: ISOLATION OF N. CANINUM cDNA AND GENE SEQUENCES

### 5.1. Identification of λ Clones Containing GRA1, GRA2, SAG1 and MIC1 cDNAs

A cDNA library of *N. caninum* tachyzoites was obtained from Dr. T. Baszler, Washington State University, Pullman, WA. Briefly, this library was constructed using RNA purified from *N. caninum* NC-1 tachyzoites. cDNAs were cloned in bacteriophage λZAPExpress (Stratagene, La Jolla, CA) following addition of *Eco*RI and *Xhol* linkers to the cDNA ends. The library was estimated to contain ~99% recombinants based on the formation of white plaques when aliquots of the library were mixed with *E. coli* XL-1 Blue MRA(P2) (Stratagene) and plated on NZY agar plates containing IPTG.

The recombinant insert DNA sequences of individual putative λZAPExpress clones identified as described above were subjected to PCR analyses essentially as described by Krishnan *et al*., 1991, Nucl. Acids. Res. 19:6177-6182; and Krishnan *et al*., 1993, Meth. Enzym. 218:258-279, which publications are incorporated herein by reference. Thus, plugs of agar containing well separated bacteriophage λ plaques were recovered using a sterile Pasteur pipette and immersed in 100 µl of sterile water for at least 1 hr. About 10 µl of the diffused bacteriophage λ. particles was used to perform PCR in a total volume of 100 µl containing: (1) 100 ng each of λDASH-T3 and λDASH-T7 oligonucleotide primers specific to the λ bacteriophage vectors, *i.e*., λZAPExpress, with specificity to the sequences adjacent to the cloning sites (*i.e*., *Eco*RI and *Xhol*), and oriented in a 5' to 3' direction towards the insert DNA sequences; (2) 200 µM dNTPs; (3) PCR buffer (Life Technologies, Inc.. Gaithersburg, MD); and (4) ~1 unit of Taq DNA polymerase buffer (Life Technologies, Inc.). The sequence of λDASH-T3 is 5'-AATTAACCCTCACTAAAGGG (SEQ ID NO:14). The sequence of λDASH-T7 is 5'-GTAATACGACTCACTATAGGGC (SEQ ID NO:15). Thermal cycling conditions were as follows: 94°C, 5 min, 1 cycle; 94°C, 1 min, 55°C, 1 min, 72°C, 1 min, 30 cycles; 72°C, 7 min, 1 cycle. An aliquot of the reaction mixture (typically 10 µl) was examined by standard agarose gel electrophoresis, ethidium bromide staining and visualization under UV illumination. The PCR mixtures were purified by ion exchange column chromatography using a PCR purification system (Qiagen), and sequenced directly using the λDASH-T3 and λDASH-T7 primers employing fluorescent labeling and the Sanger dideoxy chain termination DNA sequencing technology. Sequences were analyzed for homology to other known sequences by comparison to DNA sequence databases at the National Center for Biotechnology Information, Bethesda, Maryland, 20894, USA. Four sequences, with homology to *T. gondii GRA1*, *GRA2, SAG1* and *MIC1* genes, respectively, were identified.

### 5.2. Identification of Complete ORFs for N. caninum GRA1, GRA2, SAG1 and MIC1 cDNAs

The above-described bacteriophage λZAPExpress particles identified as containing *N*. *caninum* sequences having homology to *T. gondii GRA1*, *GRA2*, *SAG1*, and *MIC1* genes, respectively, were subjected to an *in vivo* excision protocol following manufacturer's instructions (Stratagene) to recover the insert sequences in plasmid pBluescript. Briefly, the phage particles were allowed to infect *E. coli* XL-1 Blue MRF' co-infected with ExAssist helper phage (Stratagene). Following this treatment, the supematant was collected and used to mix with *E. coli* XLOLR cells (Stratagene). Aliquots of the cell suspension were then plated on media containing kanamycin (~50 µg/ml), and kanamycin-resistant colonies were examined for plasmid profile. Plasmid DNA was purified and the recombinant portion sequenced using the Sanger dideoxy chain termination DNA sequencing technology. DNA sequences obtained were analyzed by DNASTAR (DNASTAR, Inc., Madison, WI) to identify ORFs and other features. The sequences were also analyzed using BLAST algorithms (National Center for Biotechnology Information) for homology comparison to DNA sequences in the public databases.

The recombinant plasmid clone identified as containing the complete *N. caninum GRA1* ORF was designated as pRC77 (ATCC 209685). The total length of the cDNA insert sequence in pRC77 is 1,265 bp, with the *GRA1* ORF extending from nts 205-777 (SEQ ID NO:1). The deduced amino acid sequence of the *N. caninum* GRA1 protein is presented as SEQ ID NO:2. The nucleotide sequence of the *N. caninum GRA1* ORF has ~55% similarity to the nucleotide sequence of the *T. gondii GRA1* ORF. The deduced amino acid sequence of the *N. caninum* GRA1 protein has ~51% similarity to the deduced amino acid sequence of the *T. gondii* GRA1 protein.

The recombinant plasmid clone identified as containing the complete *N. caninum GRA2* ORF was designated as pRC5 (ATCC 209686). The total length of the cDNA insert sequence in pRC5 is 1,031 bp, with the GRA2 ORF extending from nts 25-660 (SEQ ID NO:4). The deduced amino acid sequence of the *N. caninum* GRA2 protein is presented as SEQ ID NO:5. The nucleotide sequence of the *N. caninum GRA2* ORF has ~37% similarity to the nucleotide sequence of the *T. gondii GRA2* ORF. The deduced amino acid sequence of the *N. caninum* GRA2 protein has ~26% similarity to the deduced amino acid sequence of the *T. gondii* GRA2 protein.

The recombinant plasmid clone identified as containing the complete *N. caninum SAG1* ORF was designated as pRC102 (ATCC 209687). The total length of the cDNA insert sequence in pRC102 is 1,263 bp, with the *SAG1* ORF extending from nts 130-1,089 (SEQ ID NO:6). The deduced amino acid sequence of the *N. caninum* SAG1 protein is presented as SEQ ID NO:7. The nucleotide sequence of the *N. caninum SAG1* ORF has ~58% similarity to the nucleotide sequence of the *T. gondii SAG1* ORF. The deduced amino acid sequence of the *N. caninum* SAG1 protein has ~49% similarity to the deduced amino acid sequence of the *T. gondii* SAG1 protein.

The recombinant plasmid clone identified as containing the complete *N. caninum MIC1* ORF was designated as pRC340 (ATCC 209688). The total length of the cDNA insert sequence in pRC340 is 2,069 bp, with the *MIC1* ORF extending from nts 138-1.520 (SEQ ID NO:8). The deduced amino acid sequence of the *N. caninum* MIC1 protein is presented as SEQ ID NO:9. The nucleotide sequence of the *N. caninum MIC1* ORF has ~58% similarity to the nucleotide sequence of the *T. gondii MIC1* ORF. The deduced amino acid sequence of the *N. caninum* MIC1 protein has ~47% similarity to the deduced amino acid sequence of the *T. gondii* MIC1 protein.

### 5.3. Identification Of The GRA1 Gene Sequence

A genomic DNA library of *N. caninum* strain NC-1 was constructed in bacteriophage λ-II vector (Stratagene) according to conventional techniques. cDNA sequences derived from pRC77 (ATCC 209685) were PCR amplified as follows, and the resulting PCR amplified DNA fragment was used as a probe to screen the *N. caninum* strain NC-1 genomic DNA library. Primers bd219 and bd220 specific to *N. caninum GRA1* cDNA were used to amplify a 563 bp fragment corresponding to the ORF of *N. caninum GRA1* cDNA (pRC77). bd219 is 5'-GCCGCGACTTCTTTTTCTCT (SEQ ID NO:16) and bd220 is 5'-CTCGATCGCCTCCTTTACTG (SEQ ID NO:17). The 563 bp fragment was purified by electrophoresis using SeaPlaque low melting agarose (LMA) (FMC Bioproducts) The band was excised from the gel and subsequently used in random prime labeling reactions to generate a probe in preparation for screening a *Neospora* genomic library.

2.5 x 10⁵ pfu from a *N. caninum* genomic library (λDASH Stratagene #845201) were plaque-lifted onto Hybond N+ nylon membrane (Amersham). Duplicate filters were screened using the 563 bp *GRA1* cDNA fragment as a probe. Nine duplicate pfus were scored positive and subsequently cored in 1 ml SM buffer. Four of these clones (# 5-8) were carried forward to secondary screening. On secondary screening, 500-1000 pfu per clone were plaque-lifted onto duplicate filters. All four Gra1 clones were positive on secondary screening and were isolated as individual plaques.

A λ clone designated as Gra1#8 was identified by this procedure, and was used as a template for PCR amplification using primers bd256 and bd254. Primer bd256 is 5'-TGCTAGTACTGGCGAGTGAA (SEQ ID NO:18). Primer bd254 is 5'-CAGGTTTGCCACACATTTTT (SEQ ID NO:19). The PCR fragment obtained was subcloned into pGEM-T EASY vector (Promega, Madison, WI). The cloned fragment was sequenced employing fluorescent labelling and Sanger dideoxy chain termination sequencing technology. Sequence analysis revealed that the cloned fragment contained the *GRA1* gene. The *GRA1* gene sequence (SEQ ID NO:3) contains an ORF from nt 605 to nt 855 and from nt 983 to nt 1304, which shares complete identity to the *GRA1* cDNA sequence (SEQ ID NO:1) of pRC77 (ATCC 209685) from nt 205 to nt 777. However, the *GRA1* gene sequence (SEQ ID NO:3) differs from the cDNA sequence (SEQ ID NO:1) at a single nucleotide position in the 3' untranslated region at nt 1728 of the *GRA1* gene where a thymine resides, instead of a guanine at nt 1201 of pRC77. This difference may be due to a RFLP or a sequencing error in pRC77 because this nucleotide discrepancy was confirmed in 2 separate subclones from the GRA1#8 λ genomic clone. The GRA1 gene sequence (SEQ ID NO:3) further comprises an intron extending from nt 856 to nt 982. Furthermore, three promoter motifs have been identified within 150 bp 5' of the mRNA start site that are similar to those found in *T. gondii GRA* genes (Mercier *et al*., 1996, Mol. Microbiol. 21:421-428).

### 5.4. Identification Of The SAG1 Gene Sequence

Oligonucleotide primers specific to the *SAG1* gene were synthesized based on the *SAG1* ORF of the DNA sequence obtained from pRC102. The first primer, designated as NCSAG1 5', was 5'-ATGTTTCCTCCTCGGGCAGTG (SEQ ID NO:20); and the second pnmer, designated as NCSAG1 3', was 5'-TCACGCGACGCCAGCCGCTATCG (SEQ ID NO:21). It was later determined that primer NCSAG1 5', as presented above, was inadvertently designed to include an additional three nucleotides (CCT), and the presence of these three additional nucleotides was thus taken into account when determining the actual *SAG1* gene sequence

PCR was performed using primers NCSAG1 5' (SEQ ID NO:20) and NCSAG1 3' (SEQ ID NO.21) on *N. caninum* strain NC-1 genomic DNA as template. An ~1 kb amplified fragment was obtained, which was cloned in plasmid pCR2.1 and in pBlunt (Invitrogen, Carlsbad, CA) according to manufacturer's recommendations. Recombinant plasmids identified to contain the genomic *SAG1* PCR fragment were sequenced employing fluorescent labeling and Sarger dideoxy chain termination sequencing technology using standard 'universal', 'reverse' and the following oligonucleotides: NCSAG1200: 5'-GCCCTGACAATTCGACCGCC (SEQ ID NO:22); NCSAG1500: 5'-CCCACAACATCCAAGTCGTTC (SEQ ID NO.23); NCSAG1660: 5'-GTTTTGCACCATCCTTAGTG (SEQ ID NO:24); and NCSAG1320: 5'-GAGAGTTT GCTTTGCACCG (SEQ ID NO:25). The DNA sequences obtained were assembled using the DNAStar software package, and were found to be identical to the sequence of the *SAG1* ORF deduced from pRC102. Thus, the genomic sequence of the SAG1 gene is identical to that obtained from cDNA sequencing.

### 5.5. Identification Of The MIC1 Gene Sequence

A ~2.2 kb DNA fragment was PCR amplified from *N. caninum* genomic DNA using oligonucleotides specific for the 5' and 3' ends of the *MIC1* cDNA fragment (see sequence of pRC340). Thermal cycling conditions were as follows: 94°C, 1 min, 1 cycle; 94°C, 45 sec, 54°C, 45 sec, 72°C, 2 min, 29 cycles; 72°C, 5 min, 1 cycle. This -2.2 kb fragment was cloned into pCR2.1 and into pZEROBLUNT (Invitrogen, Carlsbad, CA). Recombinant plasmids were identified by standard restriction analysis, and representative clones were sequenced using fluorescent labelling and Sanger dideoxy chain termination technology. Locations of exons and introns were identified by comparison to the MIC1 cDNA sequence from pRC340.

The total length of the *MIC1* gene region is 2278 bp (SEQ ID NO:10), comprising an ORF from nt 1 to nt 73, nt 345 to nt 811, nt 1187 to nt 1265, and nt 1515 to nt 2278, with three intervening introns.

### 5.6. Identification Of The MAG1 Gene Sequence

BspDI, EcoRI and HindIII Vectorette libraries (Genosys) were prepared according to manufacturer's protocols using genomic clone Gra1#8 as template DNA. Using the antisense primer bd234 specific for 5' GRA1 cDNA, and Vectorette primer II (ER-70), a ~2 kb fragment was amplified from the HindIII Vectorette library using Klentaq (AB Peptide Inc.) and PFU (Stratagene) polymerases. Primer bd234 is 5'-CCAGCCGAGTTCGTGTTCAGA (SEQ ID NO:26). and primer ER-70 is CAACGTGGATCCGATTCAAGCTTC (SEQ ID NO:27). The product was run on a 1% LMA gel, excised, and used directly in a cloning reaction with pGEM-T EASY vector. Transformation into *E. coli* DH5α produced several white colonies. NotI restriction analysis of DNA from twenty different white clones indicated that 18 of 20 clones contained the appropriate sized insert. Subclone 2 was selected to be grown as stock and this plasmid was renamed bd245. The PCR product from the Vectorette 2 kb Gra1 promoter fragment was sequenced from both ends using nested primer bd218 and the Vectorette sequencing primer. The sequence of primer bd218 is 5'-AAAGCTCTTCGGCAGTTCAA (SEQ ID NO:28). The complete sequence of plasmid bd245 was generated by standard primer walking using Sanger fluorescent dideoxy chain termination sequencing technology.

Primer bd252 was used in combination with a variant of primer T7, and Gra1#8 DNA as template, in a PCR to map one end of clone Gra1#8. Primer bd252 is 5'-CCGCGCTACCACTTTCCA (SEQ ID NO:29). The T7 primer variant is 5'-GTAATACGACTCACTATA (SEQ ID NO:30). A ~2.5 kb fragment was amplified using primer bd252 and the T7 variant, which product was subcloned into pGEM-T EASY vector, and this plasmid was named bd282. Primer walking, using fluorescent labeling and Sanger dideoxy chain termination sequencing technology, was employed to complete the entire sequence of plasmid bd282.

Sequences from plasmids bd245 and bd282 were used to generate the contiguous sequence shown in SEQ ID NO:11, encoding the *MAG1* gene which was identified using WU-BLAST2 (Washington University BLAST version 2). Results indicate that this sequence has homology to the *T. gondii MAG1* gene (Accession No. U09029). Putative exon/intron boundaries were identified by intron splice site consensus sequences and alignment with the *T. gondii MAG1* sequence, which suggested an mRNA transcript from nt 704 to nt 820 (exon 1), from nt 1301 to nt 1399 (exon 2), from nt 1510 to nt 1808 (exon 3), and from nt 1921 to nt 3297 (exon 4), with intervening introns. Based on these putative exon/intron boundaries, a proposed cDNA sequence is presented as SEQ ID NO:12, and an amino acid sequence deduced therefrom is provided as SEQ ID NO:13. Comparison of exon and intron boundaries between *T. gondii* and *N. caninum* indicate that exons 1-3 and introns 1-2 of the *MAG1* gene are relatively positionally conserved between the two organisms. Intron 3 and exon 4 splice sites are unique to *N. caninum MAG1.* SEQ ID NO:11 also comprises a portion of the GRA1 gene sequence of *GRA1*, from nt 1 to nt 126, and the complete intervening putative bidirectional *GRA1*/*MAG1* promoter region, from nt 127 to nt 703.

DNA from lambda Gra1#8 clone was digested with NotI to release insert DNA, which was subsequently extracted with phenol/chloroform, precipitated and resuspended in water. DNA from this preparation was ligated to purified NotI digested BS KS+ vector DNA (Stratagene), and thereafter transformed into *E. coli* DH5α cells. Clones were screened by PCR using primers specific for *GRA1* and *MAG1* genes, and further verified by NotI restriction digestion for the presence of the ~16 kb lambda GRA1#8 NotI insert The primers used for PCR were *GRA1* primers 219 (SEQ ID NO:16) and 220 (SEQ ID NO:17), and *MAG1* primers 261 and 270. Primer 261 is 5'-CCGCAACGTGCTGTTCCTA (SEQ ID NO:31); and primer 270 is 5'-CATCAGAGAAACTGGAGT (SEQ ID NO:32). A positive plasmid clone containing the BS KS+ vector ligated to the NotI insert from lambda Gra1#8 was identified and named bd304 (ATCC 203413).

### 5.7. Identification Of The MAG1 And GRA1 Promoters of Neospora

### 5.7.1. Background On T. gondii GRA1 Promoter Elements

Functional mutational analysis of the *T. gondii GRA1* promoter and sequence comparison to another well-defined *T. gondii* promoter (SAG1) identified a heptanucleotide motif (TGAGACG) which confers basal *GRA1* promoter activity in an orientation-independent manner (Mercier *et al*., 1996, Mol. Micro. 21:421-428). Two additional heptanucleotide motifs in the *GRA1* promoter confer additional transcriptional activity The *T. gondii GRA1* promoter is contained within the upstream, proximal region from -129 to -47 relative to the *GRA1* transcription start site. Significant promoter elements in this *T. gondii GRA1* region include 1 CAAT box, 1 heptanucleotide motif in direct orientation and 2 heptanucleotide motifs in an inverse orientation. Three additional heptanucleotide motifs were identified upstream (-349 to - 204) of the *T. gondii GRA1* promoter but do not confer significant increase to the -129 to -47 promoter element.

### 5.7.2. Neospora MAG1-GRA1 Promoter Elements

Genomic sequence analysis of the complete *MAG1-GRA1* region of *N. caninum* strain NC-1 indicates that the two genes are arranged in a head to nead configuration. There is a 577 bp region between the putative translational start sites for the *MAG1* and *GRA1* genes (SEQ ID NO:11, from nt 127 to nt 703) that contains the putative *MAG1*/*GRA1* bi-directional promoter. Sequence analysis of this 577 bp region identifies three inverted heptanucleotide motifs (CGTCTCA or CGTCTCT) as described for the *T. gondii GRA1* promoter (Mercier *et al*., 1996. above). Two CAAT boxes flank these heptanucleotide motifs one CAAT box is oriented toward the *GRA1* gene and the second CAAT box is onented toward the *MAG1* gene. The Table below lists the promoter elements found in the *N. caninum MAG1*/*GRA1* bidirectional promoter region.

**TABLE**

| **promoter element** | **position to putative transcriptional start site defined by pRC77**^{a} |
|---|---|
| CAAT box | -133 to -130 |
| CAAT box (reverse)* | -49 to -52 |
| CGTCTCA** | -125 to -119 |
| CGTCTCA** | -106 to -100 |
| CGTCTCT** | -70 to -64 |

| | |
|---|---|
| ^{a} Nucleotide positions are in reference to the putative transcription start site defined by the 5' end of the *GRA1* cDNA (pRC77). | |
| * This CAAT box is read from the complement strand and is oriented toward the *MAG1* gene (65kDa). | |
| ** Inverted heptanucleotide promoter motifs. as defined by Mercier *et al.* 1996, above. | |

### 5.7.3. Construction Of Neospora GRA1 Promoter Construct

The functionality of the 577 bp putative *MAG1*/*GRA1* bidirectional promoter containing the two heptanucleotide motifs and two CAAT boxes was tested by engineering a plasmid containing the *LacZ* reporter gene downstream of this defined sequence and then transfecting this plasmid into NC-1 tachyzoites. A Biuescript plasmid, designated as GLS, containing the *T. gondii GRA1* promoter driving *LacZ* expression and containing a *T. gondii SAG1* 3' end, was provided by Dr. David Sibley, Washington University School of Medicine, St. Louis, MO., USA. HindIII/Nsil digestion of plasmid GLS removed the *T. gondii GRA1* promoter fragment, and subsequent LMA purification was performed to generate a promoter-less *LacZ* reporter vector. Pnmers HindIII-bd256 (5'-GGCCAAGCTTGCTAGTACTGGCGA; SEQ ID NO:33) and bd260-Nsil (5'-ATCCAATGCATCTTGCTGAATGCCTTAAAAG; SEQ ID NO:34) were used in an amplification reaction with lambda clone gra1#8 as template, and PFU and Klentaq polymerases, to generate an ~600bp promoter fragment containing the 5' untranslated region from the *Neospora GRA1* gene. This fragment was digested with HindIII/Nsil, purified on LMA, and subsequently used in a ligation reaction with the above described promoter-less *LacZ* reporter vector to generate plasmid clone bd266. A PCR reaction with primers HindIII-bd256 and bd260-Nsil was performed with plasmid clone bd266 to verify insertion of the *N. caninum GRA1* promoter.

*N. caninum* NC-1 tachyzoites (1 x 10⁷) were transfected by electroporation with 5 µg or 50 µg of uncut plasmid bd266 or plasmid GLS at 1.4V, 10 uF, in cytomix buffer as described by Howe *et al*., 1997, METHODS: A COMPANION TO METHODS IN ENZYMOLOGY 13:1-11. Electroporated NC-1 cells were allowed to infect MARC-145 monkey kidney cells in a T25 flask (80% confluency) for 3 days before harvesting for a β-galactosidase assay. Cells were harvested by removing 3 ml of media and using the remaining 1 ml of media to scrape cells from flask. Harvested cells were transferred to a microcentrifuge and spun. Supernatant was discarded, and the pelleted cells were resuspended in 100 ul of lysis buffer (Howe *et al.,* 1997, above). Tubes were stored at -20°C until the β-galactosidase assay was performed.

To conduct the β-galactosidase assay, tubes were thawed, mixed, incubated at 50°C for 1 hr, and then spun in a microcentrifuge. Fifty µl of supernatant was used per sample. The β-galactosidase assay was performed as described by Hcwe *et al*., 1997. above. A standard curve was prepared using a strain of *N. caninum* that had been stably transfected with the plasmid GLS, as provided by Dr. David Sibley. Tachyzoites were harvested, counted, resuspended in lysis buffer at 10⁴ parasites/ml, and subsequently processed as above (*i.e*., incubated at 50°C for 1 hr.). Twelve serial dilutions from this preparation were made in a range of from about 20,000 to about 10 parasites per well. and were used to create a standard curve in the β-galactosidase assay.

### 5.7.4. Results

Samples containing cell lysate from *N. caninum* strain NC-1 transfected with plasmid bd266 gave the highest β-galactosidase readings compared to samples containing cell lysate from *N. caninum* strain NC-1 transfected with plasmid GLS. Using the extracted value from the standard curve for bd266 (50 µg plasmid), the β-galactosidase reading was equivalent to 7013 parasites from the *N. caninum* cell line stably transformed with plasmid GLS described above. These experiments provide the first evidence that the *N. caninum GRA1* promoter is functional. and that the promoter elements lie within the ~600bp genomic fragment defined by primers HindIII-bd256 and bd260-Nsil.

### 6. EXAMPLE: EXPRESSION AND IMMUNOREACTIVITY OF A RECOMBINANT N. CANINUM MIC1 PROTEIN

DNA sequences representing the *MIC1* ORF were PCR-amplified and cloned into pQE50 (Qiagen), which is a recombinant system that facilitates inducible high level expression of the cloned sequence. The recombinant plasmid was designated as pQEmic1. Whole cell lysates from uninduced and induced *E. coli* cells containing pQEmic1 were examined by SDS-PAGE and Coomassie blue protein staining. A polypeptide with a molecular weight of ~57 kDa was identified in induced. but not in uninduced, *E. coli* cells carrying pQEmic1. The molecular weight of the MIC1 polypeptide as estimated from the deduced amino acid sequence of MIC1 (SEQ ID NO:9) is ~49 kDa.

Whole cell lysates of induced and uninduced *E. coli* carrying pQEmic1 were run on SDS-PAGE, and the proteins were transferred to PVF membranes (Novex) by standard procedures. The membranes were then blocked using 1% polyvinyl alcohol (PVA) in phosphate buffered saline (PBS). Following this, the membranes were rinsed three times in PBS containing 0.05% Tween-20 (PBST). The membranes were then incubated for about 1 hr either in a solution containing pooled polyclonal antisera from a naturally *N. caninum*-infected cattle herd (a gift from Dr. John Ellis, University of Technology, Sydney, Australia), or in a solution containing polyclonal antisera from rabbits experimentally infected with *T. gondii (a* gift from Dr. R. A. Cole, National Wildlife Health Center, Madison, Wl). The membranes were then washed 3x with PBST, and reacted with goat anti-bovine or anti-rabbit IgG/alkaline phosphate conjugate (Kirkegaard and Perry Labs, Gaithersburg, MD), as appropriate, diluted 1 500 according to manufacturer's recommendations. The membranes were washed in PBST again and bands were detected by incubating the membranes briefly in BCIP/NBT reagent ((Kirkegaard and Perry Labs). followed by rinsing in dH₂O. The recombinantly-expressed MIC1 protein was found to have specific reactivity to both *N*. *caninum* and *T.gondii* polyclonal antisera.

### 7. EXAMPLE: VACCINE FORMULATIONS

A vaccine against neosporosis is formulated by combining a *N. caninum* protein of the present invention, such as, *e.g*., SAG1, at 100 µg/ml with an equal volume of modified SEAM62 adjuvant, followed by gentle mixing, and storage at 4°C, for primary and boost immunizations. A primary dose of about 2 ml (total 100 µg) is administered subcutaneously to cattle, followed by a booster vaccination three weeks later. After two weeks following boost vaccination, cattle can be bred. Vaccines comprising a GRA1, GRA2, MIC1 or MAG1 protein of the present invention can also be formulated and administered in this manner.

### Deposit Of Biological Materials

The following biological materials were deposited with the American Type Culture Collection (ATCC) at 12301 Parklawn Drive, Rockville, MD, 20852, USA, on March 19, 1998, and were assigned the following accession numbers:

| Plasmid | ATCC Accession No. |
|---|---|
| pRC77 | 209685 |
| pRC5 | 209686 |
| pRC102 | 209687 |
| pRC340 | 209688 |

The following additional biological material was deposited with the ATCC, at 10801 University Blvd, Manassas, VA, 20110, USA, on November 9, 1998, and was assigned the following accession number:

| Plasmid | ATCC Accession No. |
|---|---|
| bd304 | 203413 |

All patents, patent applications, and publications cites above are incorporated herein by reference in their entirety.

The present invention is not limited in scope by the specific embodiments described. which are intended as single illustrations of individual aspects of the invention. Functionally equivalent compositions and methods are within the scope of the invention. Indeed, various modifications of the invention, in addition to those shown and described herein, will become apparent to those skilled in the art from the foregoing description. Such modifications are intended to fall within the scope of the appended claims.

## Claims

1. An isolated polynucleotide molecule comprising a nucleotide sequence encoding a *Neospora* GRA2 protein, said nucleotide sequence selected from the nucleotide sequence of the ORF of SEQ ID NO: 4 from about nt 25 to about nt 660, or the nucleotide sequence of the GRA2-encoding ORF of plasmid pRC5 (ATCC 209686).

2. The isolated polynucleotide molecule of claim 1, comprising the nucleotide sequence of SEQ ID NO: 4.

3. An isolated polynucleotide molecule comprising a nucleotide sequence that is homologous to the nucleotide sequence of a polynucleotide molecule of claim 1.

4. An isolated polynucleotide molecule comprising anucleotide sequence encoding a polypeptide that is homologous to a polypeptide comprising the amino acid sequence of SEQ ID NO: 5.

5. An isolated polynucleotide molecule consisting of a nucleotide sequence that is a substantial portion of the nucleotide sequence of the polynucleotide molecule of claim 1, 3 or 4.

6. An isolated polynucleotide molecule comprising a nucleotide sequence selected from SEQ ID NO: 4 from about nt 1 to about nt 24, SEQ ID NO: 4 from about nt 661 to about nt 1031; or a substantial portion thereof.

7. An oligonucleotide molecule selected from the group consisting of SEQ ID NOS: 14 to 26 and 28 to 34, and the complements thereof.

8. A recombinant vector, comprising a polynucleotides molecule: (a) comprising a nucleotide sequence encoding a *N. caninum* GRA2 protein; (b) comprising a nucleotide sequence that is homologous to the nucleotide sequence of (a); or (c) consisting of a nucleotide sequence that is a substantial portion of the nucleotide sequence of (a) or (b).

9. The recombinant vector of claim 8, comprising plasmid pRC5 (ATCC 209686).

10. The recombinant vector of claim 8 further comprises a polynucleotide molecule comprising a nucleotide sequence encoding a carrier or fusion partner such that expression of the recombinant vector results in production of a fusion protein comprising the carrier or fusion partner fused to the polypeptide encoded by the recombinant vector of claim 8.

11. A transformed host cell, comprising the recombinant vector of claims 8, 9 or 10.

12. A substantially purified or isolated polypeptide selected from (a) an *N*. *caninum* GRA2 protein; (b) a polypeptide having an amino acid sequence that is homologous to an *N. caninum* GRA2 protein; (c) a polypeptide consisting of a substantial portion of an *N. caninum* GRA2 protein, or polypeptide which is homologous thereto; (d) a fusion protein comprising the protein or polypeptide of (a), (b) or (c), or (e) an analog or derivative of the protein or polypeptide of (a), (b), (c) or (d).

13. The polypeptide of claim 12, wherein the GRA2 protein comprises the amino acid sequence of SEQ ID NO: 5.

14. An isolated antibody that specifically reacts to a *N. caninum* protein GRA2.

15. A genetic construct comprising a polynucleotide molecule that can be used to disable a *Neospora* gene, comprising: (a) a polynucleotide molecule having a nucleotide sequence that is otherwise the same as a nucleotide sequence encoding a GRA2 protein from *N. caninum*, or a substantial portion of said nucleotide sequence, but which nucleotide further comprises one or more disabling mutations; or (b) a polynucleotide molecule comprising a nucleotide sequence that naturally flanks *in situ* the ORF of a *Neospora* GRA2 gene, such that transformation of a *Neospora* cell with the genetic construct of (a) or (b) results in disabling of the GRA2 gene.

16. The genetic construct of claim 15, further comprising a selectable marker.

17. The genetic construct of claim 15, wherein the respective gene is disabled as the result of a homologous recombination event.

18. A *Neospora* cell that has been modified by transformation with the genetic construct of claim 15 such that the GRA2 gene has been disabled.

19. A method of preparing modified *Neospora* cells, comprising transforming *Neospora* cells with the genetic construct of claim 15, and selecting transformed cells that express a mutant phenotype of GRA2 as a result of said transformation.

20. A vaccine against neosporosis, comprising an immunologically effective amount of a component comprising: (a) a polypeptide of claim 12; (b) a polynucleotide molecule comprising a nucleotide sequence encoding the polypeptide of claim 12; or (c) modified *Neospora* cells of claim 18; and a veterinarily acceptable carrier.

21. The vaccine of claim 20, wherein the modified *Neospora* cells are live cells.

22. The vaccine of claim 20, wherein the modified *Neospora* cells are inactivated cells.

23. The vaccine of claim 20, further comprising an adjuvant or a cytokine.

24. The vaccine of claim 23, wherein the adjuvant is selected from the RBBI adjuvant system, alum, mineral gel, an oil-in-water emulsion, a water-in-oil emulsion, Block co polymer, QS-21, SAF-M, AMPHIGEN® adjuvant, saponin, Quil A, monophosphoryl lipid A, Avridine lipid-amine adjuvant, SEAM62, or SEAM1/2.

25. The vaccine of claim 20, which further comprises an immunologically effective amount of a second component that is capable of inducing a protective response against a disease or pathological condition that afflicts a mammal.

26. The vaccine of claim 25, wherein the second component is capable of inducing, or contributing to the induction of, a protective response against a pathogen selected from bovine herpes virus, bovine respiratory syncitial virus, bovine viral diarrhea virus, parainfluenza virus types I, II, or III, *Leptospira* spp., *Campylobacter* spp., *Staphylococcus aureus*, *Streptococcus agalactiae, Mycoplasma* spp., *Klebsiella* spp., *Salmonella*, spp., rotavirus, coronavirus, rabies, *Pasteurella hemolytica*, *Pasteurella multocida*, *Clostridia* spp., *Tetanus* toxoid, *E. coli*, *Cryptosporidium* spp., *Eimeria* spp., or *Trichomonas* spp.

27. A method of preparing a vaccine against neosporosis, comprising combining an immunologically effective amount of: (a) a polypeptide of claim 12, (b) a polypeptide molecule having a nucleotide sequence encoding the polypeptide of claim 12, or (c) modified *Neospora* cells of claim 18, with a veterinarily acceptable carrier.

28. A method of vaccinating a mammal against neosporosis, comprising administering to the mammal the vaccine of claim 20.

29. A kit for vaccinating a mammal against neosporosis comprising a container comprising an immunologically effective amount of (a) a polypeptide of claim 12; or (b) a polynucleotides molecule comprising a nucleotide sequence encoding the polypeptide of claim 12; or (c) modified *Neospora* cells of claim 18.

30. A kit of claim 29 further comprising a second container comprising a veterinarily acceptable carrier or diluent.
